# EUROPEAN PATENT SPECIFICATION

(11) **EP 1 592 423 B1**
(45) Date of publication and mention of the grant of the patent: **20.04.2011**
(21) Application number: 04710081.3
(22) Date of filing: 11.02.2004
(51) Int. Cl.: A61K 31/4745, A61K 31/513, A61K 31/517, A61P 35/00

(54) **COMBINATION THERAPY OF ZD6474 WITH 5-FU AND/OR CPT-11**
KOMBINATIONSTHERAPIE VON ZD6474 MIT 5-FU ODER/UND CPT-11
POLYTHERAPIE COMPRENANT DU ZD6474 AVEC DU 5-FU ET/OU DE L'OPT-11

(30) Priority: 13.02.2003 GB 0303289; 18.06.2003 GB 0314100; 10.07.2003 GB 0316184; 05.08.2003 GB 0318311
(43) Date of publication of application: 09.11.2005
(73) Proprietor: AstraZeneca AB, 151 85 Södertälje (SE)
(72) Inventor: WEDGE, Stephen Robert, c/o AstraZeneca R & D, Macclesfield, Cheshire SK10 4TG (GB); RYAN, Anderson Joseph, c/o Astrazeneca R & D, Macclesfield, Cheshire SK10 4TG (GB)
(86) International application number: PCT/GB2004/000550
(87) International publication number: WO 2004/071397

(56) References cited:
- WO-A-01/32651

## Description

The present invention relates to a combination for use in the treatment of a cancer, particularly a cancer involving a solid tumour, which comprises one of: the administration of ZD6474 in combination with 5-FU; the administration of ZD6474 in combination with CPT-11; and the administration of ZD6474 in combination with 5-FU and CPT-11; to a pharmaceutical composition comprising one of: ZD6474 and 5-FU; ZD6474 and CPT-11; and ZD6474 and 5-FU and CPT-11; to a combination product comprising one of: ZD6474 and 5-FU; ZD6474 and CPT-11; and ZD6474 and 5-FU and CPT-11, for use in a method of treatment of a human or animal body by therapy; to a kit comprising one of: ZD6474 and 5-FU; ZD6474 and CPT-11; and ZD6474 and 5-FU and CPT-11; to the use of one of: ZD6474 and 5-FU; ZD6474 and CPT-11; and ZD6474 and 5-FU and CPT-11, in the manufacture of a medicament for use in the production of an autiangiogenic and/or vascular permeability reducing effect in a warm-blooded animal such as a human which is optionally being treated with ionising radiation.

Normal angiogenesis plays an important role in a variety of processes including embryonic development, wound healing and several components of female reproductive function. Undesirable or pathological angiogenesis has been associated with disease states including diabetic retinopathy, psoriasis, cancer, rheumatoid arthritis, atheroma, Kaposi's sarcoma and haemangioma (Fan et al, 1995, Trends Pharmacol. Sci. 16: 57-66; Folkman, 1995, Nature Medicine 1: 27-31). Alteration of vascular permeability is thought to play a role in both normal and pathological physiological processes (Cullinan-Bove et al, 1993, Endocrinology 133: 829-837; Senger et al, 1993, Cancer and Metastasis Reviews, 12: 303-324). Several polypeptides with *in vitro* endothelial cell growth promoting activity have been identified including, acidic and basic fibroblast growth factors (aFGF & bFGF) and vascular endothelial growth factor (VEGF). By virtue of the restricted expression of its receptors, the growth factor activity of VEGF, in contrast to that of the FGFs, is relatively specific towards endothelial cells. Recent evidence indicates that VEGF is an important stimulator of both normal and pathological angiogenesis (Jakeman et al, 1993, Endocrinology, 133: 848-859; Kolch et al, 1995, Breast Cancer Research and Treatment, 36:139-155) and vascular permeability (Connolly et al, 1989, J. Biol. Chem 264: 20017-20024). Antagonism of VEGF action by sequestration of VEGF with antibody can result in inhibition of tumour growth (Kim et al, 1993, Nature 362: 841-844).

Receptor tyrosine kinases (RTKs) are important in the transmission of biochemical signals across the plasma membrane of cells. These transmembrane molecules characteristically consist of an extracellular ligand-binding domain connected through a segment in the plasma membrane to an intracellular tyrosine kinase domain. Binding of ligand to the receptor results in stimulation of the receptor-associated tyrosine kinase activity which leads to phosphorylation of tyrosine residues on both the receptor and other intracellular molecules. These changes in tyrosine phosphorylation initiate a signalling cascade leading to a variety of cellular responses. To date, at least nineteen distinct RTK subfamilies, defined by amino acid sequence homology, have been identified. One of these subfamilies is presently comprised by the fms-like tyrosine kinase receptor, Flt-1, the kinase insert domain-containing receptor, KDR (also referred to as Flk-1), and another fms-like tyrosine kinase receptor, Flt-4. Two of these related RTKs, Flt-1 and KDR, have been shown to bind VEGF with high affinity (De Vries et al, 1992, Science 255: 989-991; Terman et al, 1992, Biochem Biophys. Res. Comm. 1992, 187: 1579-1586). Binding of VEGF to these receptors expressed in heterologous cells has been associated with changes in the tyrosine phosphorylation status of cellular proteins and calcium fluxes.

VEGF is a key stimulus for vasculogenesis and angiogenesis. This cytokine induces a vascular sprouting phenotype by inducing endothelial cell proliferation, protease expression and migration, and subsequent organisation of cells to form a capillary tube (Keck, P.J., Hauser, S.D., Krivi, G., Sanzo, K., Warren, T., Feder, J., and Connolly, D.T., Science (Washington DC), 246: 1309-1312, 1989; Lamoreaux, W.J., Fitzgerald, M.E., Reiner, A., Hasty, K.A., and Charles, S.T., Microvasc. Res., 55: 29-42, 1998; Pepper, M.S., Montesano, R., Mandroita, S.J., Orci, L. and Vassalli, J.D., Enzyme Protein, 49: 138-162, 1996.). In addition, VEGF induces significant vascular permeability (Dvorak, H.F., Detmar, M., Claffey, K.P., Nagy, J.A., van de Water, L., and Senger, D.R., (Int. Arch. Allergy Immunol., 107: 233-235, 1995; Bates, D.O., Heald, R.I., Curry, F.E. and Williams, B. J. Physiol. (Lond.), 533: 263-272, 2001), promoting formation of a hyper-permeable, immature vascular network which is characteristic of pathological angiogenesis.

It has been shown that activation of KDR alone is sufficient to promote all of the major phenotypic responses to VEGF, including endothelial cell proliferation, migration, and survival, and the induction of vascular permeability (Meyer, M., Clauss, M., Lepple-Wienhues, A., Waltenberger, J., Augustin, H.G., Ziche, M., Lanz, C., Büttner, M., Rziha, H-J., and Dehio, C., EMBO J., 18: 363-374, 1999; Zeng, H., Sanyal, S. and Mukhopadhyay, D., J. Biol. Chem, 276: 32714-32719, 2001; Gille, H., Kowalski, J., Li, B., LeCouter, J., Moffat, B, Zioncheck, T.F., Pelletier, N. and Ferrara, N., J. Biol. Chem., 276: 3222-3230, 2001).

Quinazoline derivatives which are inhibitors of VEGF receptor tyro sine kinase are described in International Patent Application Publication Nos. WO 98/13354 and WO 01/32651. In WO 98/13354 and WO 01/32651 compounds are described which possess activity against VEGF receptor tyrosine kinase whilst possessing some activity against EGF receptor tyrosine kinase. The compound of the present invention, ZD6474, falls within the broad general disclosure of WO 98/13354 and is exemplified in WO 01/32651.

In WO 01/32651 it is stated that compounds of that invention:
"may be applied as a sole therapy or may involve, in addition to a compound of the invention, one or more other substances and/or treatments. Such conjoint treatment may be achieved by way of the simultaneous, sequential or separate administration of the individual components of the treatment." WO 01/32651 then goes on to describe examples of such conjoint treatment including surgery, radiotherapy and many types of chemotherapeutic agent including 5-fluorouracil (5-FU) and irinotecan (CPT-11). Nowhere in WO 01/32651 does it state that use of any compound of the invention therein with other treatments will produce surprisingly beneficial effects.

Unexpectedly and surprisingly we have now found that the particular compound ZD6474 used in combination with a particular selection of combination therapies, namely with one of: 5-FU; CPT-11; and 5-FU and CPT-11, produces significantly better antiangiogenic and/or vascular permeability reducing effects than any one of: ZD6474; 5-FU; CPT-11; and 5-FU and CPT-11 used alone. According to one aspect of the present invention, ZD6474 used in combination with one of: 5-FU; CPT-11; and 5-FU and CPT-11 produces significantly better anti-cancer effects than any one of: ZD6474; 5-FU; CPT-11; and 5-FU and CPT-11 used alone. According to one aspect of the present invention, ZD6474 used in combination with one of: 5-FU; CPT-11; and 5-FU and CPT-11 produces significantly better effects on solid tumours than any one of: ZD6474; 5-FU; CPT-11; and 5-FU and CPT-11 used alone. According to one aspect of the present invention, ZD6474 used in combination with one of: 5-FU; CPT-11; and 5-FU and CPT-11 produces significantly better effects in colorectal cancer than any one of: ZD6474; 5-FU; CPT-11; and 5-FU and CPT-11 used alone.

Anti-cancer effects of a combination of the present invention include, but are not limited to, anti-tumour effects, the response rate, the time to disease progression and the survival rate. Anti-tumour effects of a combination of the present invention include, but are not limited to, inhibition of tumour growth, tumour growth delay, regression of tumour, shrinkage of tumour, increased time to regrowth of tumour on cessation of treatment, slowing of disease progression. It is expected that when a combination of the present invention is administered to a warm-blooded animal such as a human, in need of treatment for cancer, with or without a solid tumour, said combination will produce an effect, as measured by, for example, one or more of: the extent of the anti-tumour effect, the response rate, the time to disease progression and the survival rate.

According to the present invention there is disclosed a combination for the production of an antiangiogenic and/or vascular permeability reducing effect in a warm-blooded animal such as a human, which comprises administering to said animal an effective amount of, 4-(4-bromo-2-fluoroanilino)-6-methoxy-7-(1-methylpiperidin-4-ylmethoxy)quinazoline, also known as ZD6474: 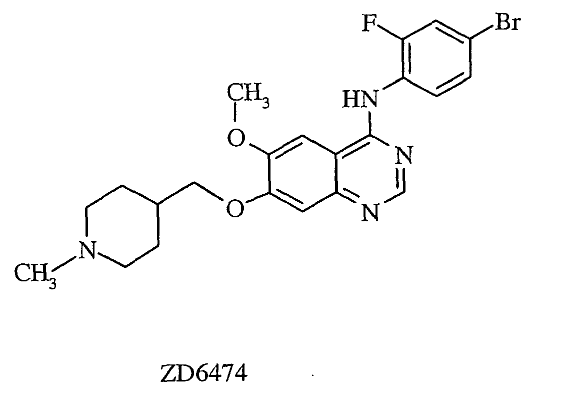 or a pharmaceutically acceptable salt thereof, before, after or simultaneously with an effective amount of one of:
a) 5-FU;
b) CPT-11; and
c) 5-FU and CPT-11.

According to a further aspect of the present invention there is provided a combination for the treatment of a cancer in a warm-blooded animal such as a human, which comprises administering to said animal an effective amount of ZD6474 or a pharmaceutically acceptable salt thereof, before, after or simultaneously with an effective amount of one of: 5-FU; CPT-11; and 5-FU and CPT-11.

According to a further aspect of the present invention there is provided a method for the treatment of a cancer involving a solid tumour in a warm-blooded animal such as a human, which comprises administering to said animal an effective amount of ZD6474 or a pharmaceutically acceptable salt thereof, before, after or simultaneously with an effective amount of one of: 5-FU; CPT-11; and 5-FU and CPT-11.

According to a further aspect of the present invention there is provided a combination for the treatment of colorectal cancer in a warm-blooded animal such as a human, which comprises administering to said animal an effective amount of ZD6474 or a pharmaceutically acceptable salt thereof, before, after or simultaneously with an effective amount of one of: 5-FU; CPT-11; and 5-FU and CPT-11.

According to a further aspect of the present invention there is provided a combination for the production of an antiangiogenic and/or vascular permeability reducing effect in a warm-blooded animal such as a human, which comprises administering to said animal an effective amount of ZD6474 or a pharmaceutically acceptable salt thereof, before, after or simultaneously with an effective amount of one of: 5-FU; CPT-11; and 5-FU and CPT-11; wherein ZD6474, 5-FU and CPT-11 may each optionally be administered together with a pharmaceutically acceptable excipient or carrier,

According to a further aspect of the present invention there is provided a combination for the treatment of a cancer in a warm-blooded animal such as a human, which comprises administering to said animal an effective amount of ZD6474 or a pharmaceutically acceptable salt thereof, before, after or simultaneously with an effective amount of one of: 5-FU; CPT-11; and 5-FU and CPT-11; wherein ZD6474, 5-FU and CPT-11 may each optionally be administered together with a pharmaceutically acceptable excipient or carrier.

According to a further aspect of the present invention there is provided a combination for the treatment of a cancer involving a solid tumour in a warm-blooded animal such as a human, which comprises administering to said animal an effective amount of ZD6474 or a pharmaceutically acceptable salt thereof, before, after or simultaneously with an effective amount of one of: 5-FU; CPT-11; and 5-FU and CPT-11; wherein ZD6474, 5-FU and CPT-11 may each optionally be administered together with a pharmaceutically acceptable excipient or carrier.

According to a further aspect of the present invention there is provided a composition for the treatment of colorectal cancer in a warm-blooded animal such as a human, which comprises administering to said animal an effective amount of ZD6474 or a pharmaceutically acceptable salt thereof, before, after or simultaneously with an effective amount of one of: 5-FU; CPT-11; and 5-FU and CPT-11; wherein ZD6474, 5-FU and CPT-11 may each optionally be administered together with a pharmaceutically acceptable excipient or carrier.

According to a further aspect of the invention there is provided a pharmaceutical composition which comprises ZD6474 or a pharmaceutically acceptable salt thereof, and 5-FU in association with a pharmaceutically acceptable excipient or carrier.

According to a further aspect of the invention there is provided a pharmaceutical composition which comprises ZD6474 or a pharmaceutically acceptable salt thereof, and CPT-11 in association with a pharmaceutically acceptable excipient or carrier.

According to a further aspect of the invention there is provided a pharmaceutical composition which comprises ZD6474 or a pharmaceutically acceptable salt thereof, and 5-FU and CPT-11 in association with a pharmaceutically acceptable excipient or carrier.

According to a further aspect of the present invention there is provided a combination product comprising ZD6474 or a pharmaceutically acceptable salt thereof and 5-FU, for use in a method of treatment of a human or animal body by therapy.

According to a further aspect of the present invention there is provided a combination product comprising ZD6474 or a pharmaceutically acceptable salt thereof and CPT-11, for use in a method of treatment of a human or animal body by therapy.

According to a further aspect of the present invention there is provided a combination product comprising ZD6474 or a pharmaceutically acceptable salt thereof and 5-FU and CPT-11, for use in a method of treatment of a human or animal body by therapy.

According to a further aspect of the present invention there is provided a kit comprising ZD6474 or a pharmaceutically acceptable salt thereof, and 5-FU.

According to a further aspect of the present invention there is provided a kit comprising ZD6474 or a pharmaceutically acceptable salt thereof, and CPT-11.

According to a further aspect of the present invention there is provided a kit comprising ZD6474 or a pharmaceutically acceptable salt thereof, and 5-FU and CPT-11.

According to a further aspect of the present invention there is provided a kit comprising:
a) ZD6474 or a pharmaceutically acceptable salt thereof in a first unit dosage form;
b) 5-FU in a second unit dosage form; and
c) container means for containing said first and second dosage forms.

According to a further aspect of the present invention there is provided a kit comprising:
a) ZD6474 or a pharmaceutically acceptable salt thereof in a first unit dosage form;
b) CPT-11 in a second unit dosage form; and
c) container means for containing said first and second dosage forms.

According to a further aspect of the present invention there is provided a kit comprising:
a) ZD6474 or a pharmaceutically acceptable salt thereof in a first unit dosage form;
b) 5-FU in a second unit dosage form;
c) CPT-11 in a third unit dosage form; and
d) container means for containing said first, second and third dosage forms.

According to a further aspect of the present invention there is provided a kit comprising:
a) ZD6474 or a pharmaceutically acceptable salt thereof, together with a pharmaceutically acceptable excipient or carrier, in a first unit dosage form;
b) 5-FU together with a pharmaceutically acceptable excipient or carrier, in a second unit dosage form; and
c) container means for containing said first and second dosage forms.

According to a further aspect of the present invention there is provided a kit comprising:
a) ZD6474 or a pharmaceutically acceptable salt thereof, together with a pharmaceutically acceptable excipient or carrier, in a first unit dosage form;
b) CPT-11 together with a pharmaceutically acceptable excipient or carrier, in a second unit dosage form; and
c) container means for containing said first and second dosage forms.

According to a further aspect of the present invention there is provided a kit comprising:
a) ZD6474 or a pharmaceutically acceptable salt thereof, together with a pharmaceutically acceptable excipient or carrier, in a first unit dosage form;
b) 5-FU together with a pharmaceutically acceptable excipient or carrier, in a second unit dosage form;
c) CPT-11 together with a pharmaceutically acceptable excipient or carrier, in a third unit dosage form; and
d) container means for containing said first, second and third dosage forms.

According to a further aspect of the present invention there is provided the use of ZD6474 or a pharmaceutically acceptable salt thereof and one of:
a) 5-FU;
b) CPT-11; and
c) 5-FU and CPT-11
in the manufacture of a medicament for use in the production of an antiangiogenic and/or vascular permeability reducing effect in a warm-blooded animal such as a human.

According to a further aspect of the present invention there is provided the use of ZD6474 or a pharmaceutically acceptable salt thereof and one of:
a) 5-FU;
b) CPT-11; and
c) 5-FU and CPT-11
in the manufacture of a medicament for use in the production of an anti-cancer effect in a warm-blooded animal such as a human.

According to a further aspect of the present invention there is provided the use of ZD6474 or a pharmaceutically acceptable salt thereof and one of:
a) 5-FU;
b) CPT-11; and
c) 5-FU and CPT-11
in the manufacture of a medicament for use in the production of an anti-tumour effect in a warm-blooded animal such as a human.

According to a further aspect of the present invention there is provided the use of ZD6474 or a pharmaceutically acceptable salt thereof and one of:
a) 5-FU;
b) CPT-11; and
c) 5-FU and CPT-11
in the manufacture of a medicament for use in the production of an anti-cancer effect m a warm-blooded animal such as a human wherein the cancer is a colorectal cancer.

According to a further aspect of the present invention there is provided a therapeutic combination treatment comprising the administration of an effective amount of ZD6474 or a pharmaceutically acceptable salt thereof, optionally together with a pharmaceutically acceptable excipient or carrier, and the administration of an effective amount of 5-FU, optionally together with a pharmaceutically acceptable excipient or carrier, to a warm-blooded animal such as a human in need of such therapeutic treatment wherein the ZD6474 and 5-FU may be administered simultaneously, sequentially or separately and in any order.

According to a further aspect of the present invention there is provided a therapeutic combination treatment comprising the administration of an effective amount of ZD6474 or a pharmaceutically acceptable salt thereof, optionally together with a pharmaceutically acceptable excipient or carrier, and the administration of an effective amount of CPT-11, optionally together with a pharmaceutically acceptable excipient or carrier, to a warm-blooded animal such as a human in need of such therapeutic treatment wherein the ZD6474 and CPT-11 may be administered simultaneously, sequentially or separately and in any order.

According to a further aspect of the present invention there is provided a therapeutic combination treatment comprising the administration of an effective amount of ZD6474 or a pharmaceutically acceptable salt thereof, optionally together with a pharmaceutically acceptable excipient or carrier, and the administration of an effective amount of 5-FU, optionally together with a pharmaceutically acceptable excipient or carrier, and the administration of an effective amount of CPT-11, optionally together with a pharmaceutically acceptable excipient or carrier, to a warm-blooded animal such as a human in need of such therapeutic treatment wherein the ZD6474, 5-FU and CPT-11 may be administered simultaneously, sequentially or separately and in any order.

A combination treatment of the present invention as defined herein may be achieved by way of the simultaneous, sequential or separate administration of the individual components of said treatment. A combination treatment as defined herein may be applied as a sole therapy or may involve surgery or radiotherapy or an additional chemotherapeutic agent in addition to a combination treatment of the invention. Surgery may comprise the step of partial or complete tumour resection, prior to, during or after the administration of the combination treatment with ZD6474 described herein.

Other chemotherapeutic agents for optional use with a combination treatment of the present invention include those described in WO 01/32651.

Such chemotherapy may cover five main categories of therapeutic agent as stated in WO 01/32651:
(i) other antiangiogenic agents including vascular targeting agents;
(ii) cytostatic agents;
(iii) biological response modifiers (for example interferon);
(iv) antibodies (for example edrecolomab); and
(v) antiproliferative/antineoplastic drugs and combinations thereof, as used in medical oncology; and other categories of agent are:
(vi) antisense therapies;
(vii) gene therapy approaches; and
(ix) immunotherapy approaches.

The administration of a multiple combination of ZD6474, 5-FU and ionising radiation or ZD6474, CPT-11 and ionising radiation or ZD6474, 5-FU, CPT-11 and ionising radiation may produce effects, such as anti-tumour effects, greater than those achieved with any of ZD6474, 5-FU, CPT-11 and ionising radiation used alone. The administration of a multiple combination of ZD6474, 5-FU and ionising radiation or ZD6474, CPT-11 and ionising radiation or ZD6474, 5-FU, CPT-11 and ionising radiation may produce effects, such as anti-tumour effects, greater than those achieved with the combination of ZD6474 and 5-FU, greater than those achieved with the combination of ZD6474 and CPT-11 and greater than those achieved with the combination of ZD6474, 5-FU and CPT-11. The administration of a multiple combination of ZD6474, 5-FU and ionising radiation or ZD6474, CPT-11 and ionising radiation or ZD6474, 5-FU, CPT-11 and ionising radiation may produce effects, such as anti-tumour effects, greater than those achieved with the combination of ZD6474 and ionising radiation, greater than those achieved with the combination of 5-FU and ionising radiation, greater than those achieved with the combination of CPT-11 and ionising radiation, and greater than those achieved with the combination of 5-FU, CPT-11 and ionising radiation.

According to the present invention there is disclosed a combination for the production of an antiangiogenic and/or vascular permeability reducing effect in a warm-blooded animal such as a human, which comprises administering to said animal an effective amount of ZD6474 or a pharmaceutically acceptable salt thereof, before, after or simultaneously with an effective amount of 5-FU and before, after or simultaneously with an effective amount of ionising radiation.

According to the present invention there is disclosed a combination for the production of an antiangiogenic and/or vascular permeability reducing effect in a warm-blooded animal such as a human, which comprises administering to said animal an effective amount of ZD6474 or a pharmaceutically acceptable salt thereof, before, after or simultaneously with an effective amount of CPT-11 and before, after or simultaneously with an effective amount of ionising radiation.

According to the present invention there is disclosed a combination for the production of an antiangiogenic and/or vascular permeability reducing effect in a warm-blooded animal such as a human, which comprises administering to said animal an effective amount of ZD6474 or a pharmaceutically acceptable salt thereof, before, after or simultaneously with an effective amount of 5-FU, before, after or simultaneously with an effective amount of CPT-11 and before, after or simultaneously with an effective amount of ionising radiation.

According to the present invention there is provided a combination for the treatment of a cancer in a warm-blooded animal such as a human, which comprises administering to said animal an effective amount of ZD6474 or a pharmaceutically acceptable salt thereof, before, after or simultaneously with an effective amount of 5-FU and before, after or simultaneously with an effective amount of ionising radiation.

According to the present invention there is provided a combination for the treatment of a cancer in a warm-blooded animal such as a human, which comprises administering to said animal an effective amount of ZD6474 or a pharmaceutically acceptable salt thereof, before, after or simultaneously with an effective amount of CPT-11 and before, after or simultaneously with an effective amount of ionising radiation.

According to the present invention there is provided a combination for the treatment of a cancer in a warm-blooded animal such as a human, which comprises administering to said animal an effective amount of ZD6474 or a pharmaceutically acceptable salt thereof, before, after or simultaneously with an effective amount of 5-FU, before, after or simultaneously with an effective amount of CPT-11 and before, after or simultaneously with an effective amount of ionising radiation.

According to the present invention there is provided a combination for the treatment of a cancer involving a solid tumour in a warm-blooded animal such as a human, which comprises administering to said animal an effective amount of ZD6474 or a pharmaceutically acceptable salt thereof, before, after or simultaneously with an effective amount of 5-FU and before, after or simultaneously with an effective amount of ionising radiation.

According to the present invention there is provided a combination for the treatment of a cancer involving a solid tumour in a warm blooded animal such as a human, which comprises administering to said animal an effective amount of ZD6474 or a pharmaceutically acceptable salt thereof, before, after or simultaneously with an effective amount of CPT-11 and before, after or simultaneously with an effective amount of ionising radiation.

According to the present invention there is provided a combination for the treatment of a cancer involving a solid tumour in a warm-blooded animal such as a human, which comprises administering to said animal an effective amount of ZD6474 or a pharmaceutically acceptable salt thereof, before, after or simultaneously with an effective amount of 5-FU, before, after or simultaneously with an effective amount of CPT-11 and before, after or simultaneously with an effective amount of ionising radiation.

In particular the cancer involving a solid tumour is colorectal cancer.

According to a further aspect of the present invention there is disclosed a combination for the production of an antiangiogenic and/or vascular permeability reducing effect in a warm-blooded animal such as a human, which comprises administering to said animal an effective amount of ZD6474 or a pharmaceutically acceptable salt thereof, before, after or simultaneously with an effective amount of 5-FU and before, after or simultaneously with an effective amount of ionising radiation, wherein ZD6474 and 5-FU may each optionally be administered together with a pharmaceutically acceptable excipient or carrier.

According to a further aspect of the present invention there is disclosed a composition for the production of an antiangiogenic and/or vascular permeability reducing effect in a warm-blooded animal such as a human, which comprises administering to said animal an effective amount of ZD6474 or a pharmaceutically acceptable salt thereof, before, after or simultaneously with an effective amount of CPT-11 and before, after or simultaneously with an effective amount of ionising radiation, wherein ZD6474 and CPT-11 may each optionally be administered together with a pharmaceutically acceptable excipient or carrier.

According to a further aspect of the present invention there is disclosed a composition for the production of an antiangiogenic and/or vascular permeability reducing effect in a warm-blooded animal such as a human, which comprises administering to said animal an effective amount of ZD6474 or a pharmaceutically acceptable salt thereof, before, after or simultaneously with an effective amount of 5-FU, before, after or simultaneously with an effective amount of CPT-11 and before, after or simultaneously with an effective amount of ionising radiation, wherein ZD6474, 5-FU and CPT-11 may each optionally be administered together with a pharmaceutically acceptable excipient or carrier.

According to a further aspect of the present invention there is provided a combination for the treatment of a cancer in a warm-blooded animal such as a human, which comprises administering to said animal an effective amount of ZD6474 or a pharmaceutically acceptable salt thereof, before, after or simultaneously with an effective amount of 5-FU and before, after or simultaneously with an effective amount of ionising radiation, wherein ZD6474 and 5-FU may each optionally be administered together with a pharmaceutically acceptable excipient or carrier.

According to a further aspect of the present invention there is provided a combination for the treatment of a cancer in a warm-blooded animal such as a human, which comprises administering to said animal an effective amount of ZD6474 or a pharmaceutically acceptable salt thereof, before, after or simultaneously with an effective amount of CPT-11 and before, after or simultaneously with an effective amount of ionising radiation, wherein ZD6474 and CPT-11 may each optionally be administered together with a pharmaceutically acceptable excipient or carrier.

According to a further aspect of the present invention there is provided a combination for the treatment of a cancer in a warm-blooded animal such as a human, which comprises administering to said animal an effective amount of ZD6474 or a pharmaceutically acceptable salt thereof, before, after or simultaneously with an effective amount of 5-FU, before, after or simultaneously with an effective amount of CPT-11 and before, after or simultaneously with an effective amount of ionising radiation, wherein ZD6474, 5-FU and CPT-11 may each optionally be administered together with a pharmaceutically acceptable excipient or carrier.

According to a further aspect of the present invention there is provided a combination for the treatment of a cancer involving a solid tumour in a warm-blooded animal such as a human, which comprises administering to said animal an effective amount of ZD6474 or a pharmaceutically acceptable salt thereof, before, after or simultaneously with an effective amount of 5-FU and before, after or simultaneously with an effective amount of ionising radiation, wherein ZD6474 and 5-FU may each optionally be administered together with a pharmaceutically acceptable excipient or carrier.

According to a further aspect of the present invention there is provided a combination for the treatment of a cancer involving a solid tumour in a warm-blooded animal such as a human, which comprises administering to said animal an effective amount of ZD6474 or a pharmaceutically acceptable salt thereof, before, after or simultaneously with an effective amount of CPT-11 and before, after or simultaneously with an effective amount of ionising radiation, wherein ZD6474 and CPT-11 may each optionally be administered together with a pharmaceutically acceptable excipient or carrier.

According to a further aspect of the present invention there is provided a combination for the treatment of a cancer involving a solid tumour in a warm-blooded animal such as a human, which comprises administering to said animal an effective amount of ZD6474 or a pharmaceutically acceptable salt thereof, before, after or simultaneously with an effective amount of 5-FU, before, after or simultaneously with an effective amount of CPT-11 and before, after or simultaneously with an effective amount of ionising radiation, wherein ZD6474, 5-FU and CPT-11 may each optionally be administered together with a pharmaceutically acceptable excipient or carrier.

According to a further aspect of the present invention there is provided the use of ZD6474 or a pharmaceutically acceptable salt thereof and 5-FU in the manufacture of a medicament for use in the production of an antiangiogenic and/or vascular permeability reducing effect in a warm-blooded animal such as a human which is being treated with ionising radiation.

According to a further aspect of the present invention there is provided the use of ZD6474 or a pharmaceutically acceptable salt thereof and CPT-11 in the manufacture of a medicament for use in the production of an antiangiogenic and/or vascular permeability reducing effect in a warm-blooded animal such as a human which is being treated with ionising radiation.

According to a further aspect of the present invention there is provided the use of ZD6474 or a pharmaceutically acceptable salt thereof and 5-FU and CPT-11 in the manufacture of a medicament for use in the production of an antiangiogenic and/or vascular permeability reducing effect in a warm-blooded animal such as a human which is being treated with ionising radiation.

According to a further aspect of the present invention there is provided the use of ZD6474 or a pharmaceutically acceptable salt thereof and 5-FU in the manufacture of a medicament for use in the production of an anti-cancer effect in a warm-blooded animal such as a human which is being treated with ionising radiation.

According to a further aspect of the present invention there is provided the use of ZD6474 or a pharmaceutically acceptable salt thereof and CPT-11 in the manufacture of a medicament for use in the production of an anti-cancer effect in a warm-blooded animal such as a human which is being treated with ionising radiation.

According to a further aspect of the present invention there is provided the use of ZD6474 or a pharmaceutically acceptable salt thereof and 5-FU and CPT-11 in the manufacture of a medicament for use in the production of an anti-cancer effect in a warm-blooded animal such as a human which is being treated with ionising radiation.

In particular the warm-blooded animal such as a human has colorectal cancer.

According to a further aspect of the present invention there is provided the use of ZD6474 or a pharmaceutically acceptable salt thereof and 5-FU in the manufacture of a medicament for use in the production of an anti-tumour effect in a warm-blooded animal such as a human which is being treated with ionising radiation.

According to a further aspect of the present invention there is provided the use of ZD6474 or a pharmaceutically acceptable salt thereof and CPT-11 in the manufacture of a medicament for use in the production of an anti-tumour effect in a warm-blooded animal such as a human which is being treated with ionising radiation.

According to a further aspect of the present invention there is provided the use of ZD6474 or a pharmaceutically acceptable salt thereof and 5-FU and CPT-11 in the manufacture of a medicament for use in the production of an anti-tumour effect in a warn-blooded animal such as a human which is being treated with ionising radiation.

According to a further aspect of the present invention there is provided a therapeutic combination treatment comprising the administration of an effective amount of ZD6474 or a pharmaceutically acceptable salt thereof, optionally together with a pharmaceutically acceptable excipient or carrier, and the administration of an effective amount of 5-FU, optionally together with a pharmaceutically acceptable excipient or carrier and the administration of an effective amount of ionising radiation, to a warm-blooded animal such as a human in need of such therapeutic treatment wherein the ZD6474, 5-FU and ionising radiation may be administered simultaneously, sequentially or separately and in any order.

According to a further aspect of the present invention there is provided a therapeutic combination treatment comprising the administration of an effective amount of ZD6474 or a pharmaceutically acceptable salt thereof, optionally together with a pharmaceutically acceptable excipient or carrier, and the administration of an effective amount of CPT-11, optionally together with a pharmaceutically acceptable excipient or carrier and the administration of an effective amount of ionising radiation, to a warm-blooded animal such as a human in need of such therapeutic treatment wherein the ZD6474, CPT-11 and ionising radiation may be administered simultaneously, sequentially or separately and in any order.

According to a further aspect of the present invention there is provided a therapeutic combination treatment comprising the administration of an effective amount of ZD6474 or a pharmaceutically acceptable salt thereof, optionally together with a pharmaceutically acceptable excipient or carrier, and the administration of an effective amount of 5-FU, optionally together with a pharmaceutically acceptable excipient or carrier, and the administration of an effective amount of CPT-11, optionally together with a pharmaceutically acceptable excipient or carrier, and the administration of an effective amount of ionising radiation, to a warm-blooded animal such as a human in need of such therapeutic treatment wherein the ZD6474, 5-FU, CPT-11 and ionising radiation may be administered simultaneously, sequentially or separately and in any order.

A warm-blooded animal such as a human which is being treated with ionising radiation means a warm-blooded animal such as a human which is treated with ionising radiation before, after or at the same time as the administration of a medicament or combination treatment comprising ZD6474 and one of: 5-FU; CPT-11; and 5-FU and CPT-11. For example said ionising radiation may be given to said warm-blooded animal such as a human within the period of a week before to a week after the administration of a medicament or combination treatment comprising ZD6474 and one of: 5-FU; CPT-11; and 5-FU and CPT-11. This means that ZD6474, 5-FU, CPT-11 and ionising radiation may be administered separately or sequentially in any order, or may be administered simultaneously. The warm-blooded animal may experience the effect of each of ZD6474, 5-FU, CPT-11 and radiation simultaneously.

According to one aspect of the present invention the ionising radiation is administered before one of ZD6474 and one of: 5-FU; CPT-11; and 5-FU and CPT-11, or after one of ZD6474 and one of: 5-FU; CPT-11; and 5-FU and CPT-11.

According to one aspect of the present invention the ionising radiation is administered before any of ZD6474 and one of: 5-FU; CPT-11; and 5-FU and CPT-11 or after all of ZD6474 and one of: 5-FU; CPT-11; and 5-FU and CPT-11.

According to one aspect of the present invention ZD6474 is administered to a warm blooded animal after the animal has been treated with ionising radiation.

As stated above the combination treatments of the present invention, that is ZD6474, optionally with ionising radiation, combined with one of: 5-FU; CPT-11; and 5-FU and CPT-11, as defined herein, are of interest for their antiangiogenic and/or vascular permeability effects. Angiogenesis and/or increased vascular permeability is present in a wide range of disease states including cancer (including leukaemia, multiple myeloma and lymphoma), diabetes, psoriasis, rheumatoid arthritis, Kaposi's sarcoma, haemangioma, acute and chronic nephropathies, atheroma, arterial restenosis, autoimmune diseases, acute inflammation, lymphoedema, excessive scar formation and adhesions, endometriosis, dysfunctional uterine bleeding and ocular diseases with retinal vessel proliferation (including age-related macular degeneration). Combination treatments of the present invention are expected to be particularly useful in the prophylaxis and treatment of diseases such as cancer and Kaposi's sarcoma. In particular combination treatments of the invention are expected to slow advantageously the growth of primary and recurrent solid tumours of, for example, the colon, breast, prostate, lungs and skin. More particularly such combination treatments of the invention are expected to inhibit any form of cancer associated with VEGF including leukaemia, mulitple myeloma and lymphoma and also, for example, to inhibit the growth of those primary and recurrent solid tumours which are associated with VEGF, especially those tumours which are significantly dependent on VEGF for their growth and spread, including for example, certain tumours of the colon, breast, prostate, lung, vulva and skin.

According to one aspect of the present invention such combination treatments of the invention are expected to slow advantageously the growth of primary and secondary (recurrent) tumours in colorectal cancer.

In another aspect of the present invention ZD6474, optionally with ionising radiation, and one of: 5-FU; CPT-11; and 5-FU and CPT-11 are expected to inhibit the growth of those primary and recurrent solid tumours which are associated with VEGF especially those tumours which are significantly dependent on VEGF for their growth and spread.

In another aspect of the present invention ZD6474, optionally with ionising radiation, and one of: 5-FU; CPT-11; and 5-FU and CPT-11 are expected to inhibit the growth of those primary and recurrent solid tumours which are associated with both VEGF and EGF especially those tumours which are significantly dependent on VEGF and EGF for their growth and spread.

According to another aspect of the present invention the effect of a combination of treatment of the present invention is expected to be at least equivalent to the addition of the effects of each of the components of said treatment used alone, that is, of each of ZD6474, 5-FU, CPT-11 and ionising radiation used alone.

According to another aspect of the present invention the effect of a combination of treatment of the present invention is expected to be greater than the addition of the effects of each of the components of said treatment used alone, that is, of each of ZD6474, 5-FU, CPT-11and ionising radiation used alone.

According to another aspect of the present invention the effect of a combination of treatment of the present invention is expected to be a synergistic effect.

According to the present invention a combination treatment is defined as affording a synergistic effect if the effect is therapeutically superior, as measured by, for example, the extent of the response, the response rate, the time to disease progression or the survival period, to that achievable on dosing one or other of the components of the combination treatment at its conventional dose. For example, the effect of the combination treatment is synergistic if the effect is therapeutically superior to the effect achievable with ZD6474, 5-FU, CPT-11, 5-FU and CPT-11, or ionising radiation used alone. Further, the effect of the combination treatment is synergistic if a beneficial effect is obtained in a group of patients that does not respond (or responds poorly) to ZD6474, 5-FU, CPT-11, 5-FU and CPT-11, or ionising radiation used alone. In addition, the effect of the combination treatment is defined as affording a synergistic effect if one of the components is dosed at its conventional dose and the other component(s) is/are dosed at a reduced dose and the therapeutic effect, as measured by, for example, the extent of the response, the response rate, the time to disease progression or the survival period, is equivalent to that achievable on dosing conventional amounts of the components of the combination treatment. In particular, synergy is deemed to be present if the conventional dose of ZD6474, 5-FU, CPT-11, 5-FU and CPT-11, or ionising radiation may be reduced without detriment to one or more of the extent of the response, the response rate, the time to disease progression and survival data, in particular without detriment to the duration of the response, but with fewer and/or less troublesome side-effects than those that occur when conventional doses of each component are used.

The compositions described herein may be in a form suitable for oral administration, for example as a tablet or capsule, for nasal administration or administration by inhalation, for example as a powder or solution, for parenteral injection (including intravenous, subcutaneous, intramuscular, intravascular or infusion) for example as a sterile solution, suspension or emulsion, for topical administration for example as an ointment or cream, for rectal administration for example as a suppository or the route of administration may be by direct injection into the tumour or by regional delivery or by local delivery. In other embodiments of the present invention the ZD6474 of the combination treatment may be delivered endoscopically, intratracheally, intralesionally, percutaneously, intravenously, subcutaneously, intraperitoneally or intratumourally. In general the compositions described herein may be prepared in a conventional manner using conventional excipients. The compositions of the present invention are advantageously presented in unit dosage form.

ZD6474 will normally be administered to a warm-blooded animal at a unit dose within the range 10-500mg per square metre body area of the animal, for example approximately 0.3-15mg/kg in a human. A unit dose in the range, for example, 0.3-15mg/kg, preferably 0.5-5mg/kg is envisaged and this is normally a therapeutically-effective dose. A unit dosage form such as a tablet or capsule will usually contain, for example 25-500mg of active ingredient. Preferably a daily dose in the range of 0.5-5mg/kg is employed.

CPT-11 is also known as irinotecan. CPT-11 may be administered in accordance with any known route of administration and dosage.

For example CPT-11 may be dosed at 350mg/m²as an intravenous infusion over a 30 to 90 minute period every 3 weeks.

CPT-11 is a semi-synthetic derivative of camptothecin and is metabolised *in vivo* to an active metabolite SN-38.

5-FU is 5-fluorouracil. 5-FU may be administered according to any known route of administration and dosage.

For example 5-FU may be given as an intravenous daily infusion of 15mg/kg diluted in 500ml of 5% dextrose solution or 500ml 0.9% sodium chloride solution given by intravenous infusion: at the rate of 40 drops per minute over 4 hours; or infused over 30 to 60 minutes; or as a daily continuous infusion over 24 hours. The daily dose of 5-FU is recommended not to exceed 1g. 5-FU is usually given daily in one of these ways until 12-15g has been given and this constitutes one course of 5-FU. It is usual practice to leave 4 to 6 weeks between courses of 5-FU. Alternatively 5-FU may be dosed by intravenous injection at a dose of 12mg/kg on three consecutive days, followed by 6mg/kg on days 5, 7 and 9 ie on the three following alternate days, followed by a maintenance dose of 5-15mg/kg by intravenous injection once a week. Alternatively 5-FU may be given by intravenous injection at a dose of 15mg/kg once a week for the duration of the patient's treatment. 5-FU may also be dosed intra-arterially as a regional perfusion at 5-7.5mg/kg by 24 hour continuous infusion. 5-FU may also be dosed orally at a dose of 15mg/kg once a week or at a dose of 15mg/kg for six successive days followed by 15mg/kg once a week.

5-FU is commonly administered with leucovorin. For the avoidance of doubt the combination treatments of the present invention include the use of 5-FU when given with, or without, leucovorin.

Leucovorin may be administered according to any known route of administration and dosage. For example leucovorin may be administered orally. When used in combination with 5-FU, leucovorin is conveniently administered as calcium leucovorin and given intravenously. For example, calcium leucovorin may be given at a dose of 200mg/m² by slow intravenous injection, followed immediately by 5-FU at an initial dose of 370mg/m² by intravenous injection. The injection of leucovorin should not be given more rapidly than over 3-5 minutes because of the calcium content of the solution. This treatment is repeated daily for 5 consecutive days. Subsequent courses may be given after a treatment-free interval of 21-28 days.

Alternatively the following regimen may be used: leucovorin 500 mg/m² given by 2 hour infusion every week for 6 weeks with 5-FU 500 mg/m² given as an iv bolus midway through the 6-week period.

Alternatively the following regimen may be used: leucovorin 200 mg/m² given by iv 2 hour infusion followed by 5-FU 400 mg/m² iv bolus followed by 5-FU 600 mg/m² given by iv 22 hour infusion, repeated for 2 consecutive days. The cycle is repeated every 2 weeks.

Alternatively 5-FU may be administered orally as capecitabine (Xeloda^{™}), tegafur, or TS-1. Capecitabine is a relatively non-cytotoxic fluoropyrimidine carbamate which functions as an orally administered precursor of 5-FU. Capecitabine may be administered according to any known dosage. For example a dose of 1250 mg/m² may be given orally twice a day, (equivalent to a daily dose of 2500mg/m²), for 14 days followed by a rest period of 7 days.

Combination treatments of the present invention include the use of 5-FU when given in any form, (including prodrug and precursor forms that are converted to 5-FU systemically or within the tumour), when administered via any route and when given with, or without, leucovorin.

Combination treatments of the present invention include the use of CPT-11 or SN-38 when given in any form, (including prodrug and precursor forms that are converted to SN-38 systemically or within the tumour and including liposomal formulations), when administered via any route.

Radiotherapy may be administered according to the known practices in clinical radiotherapy. The dosages of ionising radiation will be those known for use in clinical radiotherapy. The radiation therapy used will include for example the use of y-rays, X-rays, and/or the directed delivery of radiation from radioisotopes. Other forms of DNA damaging factors are also included in the present invention such as microwaves and UV-irradiation. For example X-rays may be dosed in daily doses of 1.8-2.0Gy, 5 days a week for 5-6 weeks. Normally a total fractionated dose will lie in the range 45-60Gy. Single larger doses, for example 5-10Gy may be administered as part of a course of radiotherapy. Single doses may be administered intraoperatively. Hyperfractionated radiotherapy may be used whereby small doses of X-rays are administered regularly over a period of time, for example 0.1Gy per hour over a number of days. Dosage ranges for radioisotopes vary widely, and depend on the half life of the isotope, the strength and type of radiation emitted, and on the uptake by cells.

As stated above the size of the dose of each therapy which is required for the therapeutic or prophylactic treatment of a particular disease state will necessarily be varied depending on the host treated, the route of administration and the severity of the illness being treated. Accordingly the optimum dosage may be determined by the practitioner who is treating any particular patient. For example, it may be necessary or desirable to reduce the above-mentioned doses of the components of the combination treatments in order to reduce toxicity.

The combination treatments of the present invention comprise: ZD6474 and 5-FU; ZD6474 and CPT-11; ZD6474, 5-FU and CPT-11; ZD6474, 5-FU and ionising radiation; ZD6474, CPT-11 and ionising radiation; ZD6474, 5-FU, CPT-11 and ionising radiation. The agents therein may be administered separately or sequentially in any order, or may be administered simultaneously.

The present invention comprises combinations of 5-FU or CPT-11 or 5-FU and CPT-11 with ZD6474 or with a salt of ZD6474.

Salts for use in pharmaceutical compositions will be pharmaceutically acceptable salts, but other salts may be useful in the production of ZD6474 and its pharmaceutically acceptable salts. Such salts may be formed with an inorganic or organic base which affords a pharmaceutically acceptable cation. Such salts with inorganic or organic bases include for example an alkali metal salt, such as a sodium or potassium salt, an alkaline earth metal salt such as a calcium or magnesium salt, an ammonium salt or for example a salt with methylamine, dimethylamine, trimethylamine, piperidine, morpholine or tris-(2-hydroxyethyl) amine.
ZD6474 may be made, for example, according to any of the following processes illustrated by examples (a) -(c) in which, unless otherwise stated:-
(i) evaporations were carried out by rotary evaporation in vacuo and work-up procedures were carried out after removal of residual solids such as drying agents by filtration;
(ii) operations were carried out at ambient temperature, that is in the range 18-25°C and under an atmosphere of an inert gas such as argon;
(iii) column chromatography (by the flash procedure) and medium pressure liquid chromatography (MPLC) were performer on Merck Kieselgel silica (Art. 9385) or Merck Lichroprep RP-18 (Art. 9303) reversed-phase silica obtained from E. Merck, Darmstadt, Germany;
(iv) yields are given for illustration only and are not necessarily the maximum attainable;
(v) melting points are uncorrected and were determined using a Mettler SP62 automatic melting point apparatus, an oil-bath apparatus or a Koffler hot plate apparatus.
(vi) the structures of the end-products of the formula I were confirmed by nuclear (generally proton) magnetic resonance (NMR) and mass spectral techniques; proton magnetic resonance chemical shift values were measured on the delta scale and peak multiplicities are shown as follows: s, singlet; d, doublet; t, triplet; m, multiplet; br, broad; q, quartet; NMR spectra were run on a 400MHz machine at 24°C.
(vii) intermediates were not generally fully characterised and purity was assessed by thin layer chromatography (TLC), high-performance liquid chromatography (HPLC), infra-red (IR) or NMR analysis;
(viii) the following abbreviations have been used:-
   DMF N,N-dimethylformamide
   DMSO dimethylsulphoxide
   THF tetrahydrofuran
   TFA trifluoroacetic acid
   NMP 1-methyl-2-pyrrolidione.]

### Process (a)

A solution of 37% aqueous formaldehyde (50µl, 0.6mmol) followed by sodium cyanoborohydride (23mg, 0.36mmol) were added to a solution of 4-(4-bromo-2-fluoroanilino)-6-methoxy-7-(piperidin-4-ylmethoxy)quinazoline (139mg, 0.3mnol), in a mixture of THF/methanol (1.4ml/1.4ml). After stirring for 1 hour at ambient temperature, water was added and the volatiles were removed under vacuum The residue was triturated with water, filtered, washed with water, and dried under vacuum. The solid was purified by chromatography on neutral alumina eluting with methylene chloride followed by methylene chloride/ethyl acetate (1/1) followed by methylene chloride/ethyl acetate/methanol (50/45/5). The fractions containing the expected product were evaporated under vacuum The resulting white solid was dissolved in methylene chloride/methanol (3ml/3ml) and 3N hydrogen chloride in ether (0.5ml) was added. The volatiles were removed under vacuum. The solid was triturated with ether, filtered, washed with ether and dried under vacuum to give 4-(4-bromo-2-fluoroanilino)-6-methoxy-7-(1-methylpiperidin-4-ylmethoxy)quinazoline hydrochloride (120mg, 69%).
MS - ESI: 475-477 [MH]⁺

The NMR spectrum of the protonated form of 4-(4-bromo-2-fluoroanilino)-6-methoxy-7-(1-methylpiperidin-4-ylmethoxy)quinazoline hydrochloride shows the presence of 2 forms A and B in a ratio A: B of approximately 9:1.
¹H NMR Spectrum: (DMSOd₆; CF₃COOD) 1.55-1.7 (m, form A 2H) ; 1.85-2.0 (m, form B 4H) ; 2.03 (d, form A 2H) ; 2.08-2.14 (br s, form A 1H) ; 2.31-2.38 (br s, form B 1H) ; 2.79 (s, form A 3H) ; 2.82 (s, form B 3H) ; 3.03 (t, form A 2H) ; 3.21 (br s, form B 2H) ; 3.30 (br s, form B 2H) ; 3.52 (d, form A 2H) ; 4.02 (s, 3H) ; 4.12 (d, form A 2H) ; 4.30 (d, form B 2H) ; 7.41 (s, 1H) ; 7.5-7.65 (m, 2H) ; 7.81 (d, 1H) ; 8.20 (s, 1H) ; 8.88 (s, 1H)

| | | | | |
|---|---|---|---|---|
| Elemental analysis: | Found | C 46.0 | H 5.2 | N 9.6 |
| C₂₂H₂₄N₄O₂BrF 0.3H₂O 2.65HCl | Requires | C 45.8 | H 4.8 | N 9.7% |

The starting material was prepared as follows:
A solution of 7-benzyloxy-4-chloro-6-methoxyquinazoline hydrochloride (8.35g, 27.8mmol), (prepared, for example, as described in WO 97/22596, Example 1), and 4-bromo-2-fluoroaniline (5.65g, 29.7mmol) in 2-propanol (200ml) was heated at reflux for 4 hours. The resulting precipitate was collected by filtration, washed with 2-propanol and then ether and dried under vacuum to give 7-benzyloxy-4-(4-bromo-2-fluoroanilino)-6-methoxyquinazoline hydrochloride (9.46g, 78%).
¹H NMR Spectrum: (DMSOd₆; CD₃COOD) 4.0(s, 3H); 5.37(s, 2H); 7.35-7.5(m, 4H); 7.52-7.62(m, 4H); 7.8(d, 1H); 8.14(9s, 1H); 8.79(s, 1H)
MS - ESI: 456 [MH]⁺

| | | | | |
|---|---|---|---|---|
| Elemental analysis: | Found | C 54.0 | H 3.7 | N 8.7 |
| C₂₂H₁₇N₃O₂BrF 0.9HCl | Requires | C 54.2 | H 3.7 | N 8.6% |

A solution of 7-benzyloxy-4-(4-bromo-2-fluoroanilino)-6-methoxyquinazoline hydrochloride (9.4g, 19. 1mmol) in TFA (90ml) was heated at reflux for 50 minutes. The mixture was allowed to cool and was poured on to ice. The resulting precipitate was collected by filtration and dissolved in methanol (70ml). The solution was adjusted to pH9-10 with concentrated aqueous ammonia solution. The mixture was concentrated to half initial volume by evaporation. The resulting precipitate was collected by filtration, washed with water and then ether, and dried under vacuum to give 4-(4-bromo-2-fluoroanilino)-7-hydroxy-6-methoxyquinazoline (5.66g, 82%).
¹H NMR Spectrum: (DMSOd₆; CD₃COOD) 3.95(s, 3H); 7.09(s, 1H); 7.48(s, 1H); 7.54(t, 1H); 7.64(d, 1H); 7.79(s, 1H); 8.31(s, 1H)
MS - ESI: 366 [MH]⁺

| | | | | |
|---|---|---|---|---|
| Elemental analysis: | Found | C 49.5 | H 3.1 | N 11.3 |
| C₁₅H₁₁N₃O₂BrF | Requires | C 49.5 | H 3.0 | N 11.5% |

While maintaining the temperature in the range 0-5°C, a solution of di-*tert-*butyl dicarbonate (41.7g, 0.19mol) in ethyl acetate (75ml) was added in portions to a solution of ethyl 4-piperidinecarboxylate (30g, 0.19mol) in ethyl acetate (150ml) cooled at 5°C. After stirring for 48 hours at ambient temperature, the mixture was poured onto water (300ml). The organic layer was separated, washed successively with water (200ml), 0.1N aqueous hydrochloric acid (200ml), saturated sodium hydrogen carbonate (200ml) and brine (200ml), dried (MgSO₄) and evaporated to give ethyl 4-(1-(*tert-*butoxycarbonyl)piperidine)carboxylate (48g, 98%).
¹H NMR Spectrum: (CDCl₃) 1.25(t, 3H); 1.45(s, 9H); 1.55-1.70(m, 2H); 1.8-2.0(d, 2H); 2.35-2.5(m, 1H); 2.7-2.95(t, 2H); 3.9-4.1(br s, 2H); 4.15 (q, 2H)

A solution of 1M lithium aluminium hydride in THF (133ml, 0.133mol) was added in portions to a solution of ethyl 4-(1-(*tert-*butoxycarbonyl)piperidine)carboxylate (48g, 0.19mol) in dry THF (180ml) cooled at 0°C. After stirring at 0°C for 2 hours, water (30ml) was added followed by 2N sodium hydroxide (10ml). The precipitate was removed by filtration through diatomaceous earth and washed with ethyl acetate. The filtrate was washed with water, brine, dried (MgSO₄) and evaporated to give 1-(*tert-*butoxycarbonyl)-4-hydroxymethylpiperidine (36.3g, 89%).
MS (EI): 215 [M.]+
¹H NMR Spectrum:: (CDCl₃) 1.05-1.2(m, 2H); 1.35-1.55(m, 10H); 1.6-1.8(m, 2H); 2.6-2.8(t, 2H); 3.4-3.6(t, 2H); 4.0-4.2(br s, 2H)

1,4-Diazabicyclo[2.2.2]octane (42.4g, 0.378mol) was added to a solution of 1-(*tert-*butoxycarbonyl)-4-hydroxymethylpiperidine (52.5g, 0.244mol) in *tert-*butyl methyl ether (525my). After stirring for 15 minutes at ambient temperature, the mixture was cooled to 5°C and a solution of toluene sulphonyl chloride (62.8g, 0.33mmol) in *tert-*butyl methyl ether (525ml) was added in portions over 2 hours while maintaining the temperature at 0°C. After stirring for 1 hour at ambient temperature, petroleum ether (11) was added. The precipitate was removed by filtration. The filtrate was evaporated to give a solid. The solid was dissolved in ether and washed successively with 0.5N aqueous hydrochloric acid (2x500ml), water, saturated sodium hydrogen carbonate and brine, dried (MgSO₄) and evaporated to give 1-(*tert-*butoxycarbonyl)-4-(4-methylphenylsulphonyloxymethyl)piperidine (76.7g, 85%).
MS (ESI): 392 [MNa]⁺
¹H NMR Spectrum: (CDCl₃) 1.0-1.2(m, 2H); 1.45(s, 9H); 1.65(d, 2H); 1.75-1.9(m, 2H); 2.45(s, 3H); 2.55-2.75(m, 2H); 3.85(d, 1H); 4.0-4.2(br s, 2H); 7.35(d, 2H); 7.8(d, 2H)

Potassium carbonate (414mg, 3mmol) was added to a suspension of 4-(4-bromo-2-fluoroanilino)-7-hydroxy-6-methoxyquinazoline (546mg, 1.5mmol) in DMF (5ml). After stirring for 10 minutes at ambient temperature, 1-(*tert-*butoxycarbonyl)-4-(4-methylphenylsulphonyloxymethyl)piperidine (636mg, 1.72mmol) was added and the mixture was heated at 95°C for 2 hours. After cooling, the mixture was poured onto cooled water (20ml). The precipitate was collected by filtration, washed with water, and dried under vacuum to give 4-(4-bromo-2-fluoroanilino)-7-(1-(*tert-*butoxycarbonyl)piperidin-4-ylmethoxy)-6-methoxyquinazoline (665mg, 79%).
MS - ESI: 561-563 [MH]⁺
¹H NMR Spectrum: (DMSOd₆) 1.15-1.3 (m, 2H), 1.46 (s, 9H), 1.8 (d, 2H), 2.0-2.1 (m, 1H), 2.65-2.9 (m, 2H), 3.95 (s, 3H), 4.02 (br s, 2H), 4.05 (d, 2H), 7.2 (s, 1H), 7.48 (d, 1H), 7.55 (t, 1H), 7.65 (d, 1H), 7.8 (s, 1H), 8.35 (s, 1H), 9.55 (br s, 1H)

TFA (3ml) was added to a suspension of 4-(4-bromo-2-fluoroanilino)-7-(1-(*tert-*butoxycarbonyl)piperidin-4-ylmethoxy)-6-methoxyquinazoline (673mg, 1.2mmol) in methylene chloride (10ml). After stirring for 1 hour at ambient temperature, the volatiles were removed under vacuum The residue was triturated with a mixture of water/ether. The organic layer was separated. The aqueous layer was washed again with ether. The aqueous layer was adjusted to pH10 with 2N aqueous sodium hydroxide. The aqueous layer was extracted with methylene chloride. The organic layer was dried (MgSO₄) and the solvent was removed under vacuum The solid was triturated with a mixture ether/petroleum ether (1/1), filtered, washed with ether and dried under vacuum to give 4-(4-bromo-2-fluoroanilino)-6-methoxy-7-(piperidin-4-ylmethoxy)quinazoline (390mg, 70.5%).
MS - ESI: 461-463 [MH]⁺
¹H NMR Spectrum: (DMSOd₆) 1.13-1.3 (m, 2H), 1.75 (d, 2H), 1.87-2.0 (m, 1H), 2.5 (d, 2H), 3.0 (d, 2H), 3.96 (s, 3H), 3.98 (d, 2H), 7.2 (s, 1H), 7.5 (dd, 1H), 7.55 (t, 1H), 7.68 (dd, 1H), 7.80 (s, 1H), 8.36 (s, 1H), 9.55 (br s, 1H)

| | | | | |
|---|---|---|---|---|
| Elemental analysis: | Found | C 54.5 | H 4.9 | N 12.1 |
| C₂₁H₂₂N₄O₂BrF | Requires | C 54.7 | H 4.8 | N 12.1% |

### Process (b)

37% Aqueous formaldehyde (3.5ml, 42mmol) was added to a solution of 4-(4-bromo-2-fluoroanilino)-7-(1-(*tert-*butoxycarbonyl)piperidin-4-ylmethoxy)-6-methoxyquinazoline (3.49g, 6.22mmol), (prepared as described for the starting material in process (a) above), in formic acid (35ml). After heating at 95°C for 4 hours the volatiles were removed under vacuum The residue was suspended in water and the mixture was adjusted to pH10.5 by slow addition of a solution of 2N sodium hydroxide. The suspension was extracted with ethyl acetate. The organic layer was washed with brine, dried MgSO₄ and evaporated to give 4-(4-bromo-2-fluoroanilino)-6-methoxy-7-(1-methylpiperidin-4-ylmethoxy)quinazoline (2.61g, 88%).
MS - ESI: 475-477 [MH]⁺
¹H NMR Spectrum: (DMSOd₆) 1.3-1.45 (m, 2H), 1.8 (d, 2H), 1.7-1.9 (m, 1H), 1.95 (t, 2H), 2.2 (s, 3H), 2.85 (d, 2H), 3.96 (s, 3H), 4.05 (d, 2H), 7.19 (s, 1H), 7.5 (d, 1H), 7.55 (t, 1H), 7.67 (d, 1H), 7.81 (s, 1H), 8.37 (s, 1H), 9.54 (s, 1H)

| | | | | |
|---|---|---|---|---|
| Elemental analysis: | Found | C 55.4 | H 5.1 | N 11.6 |
| C₂₂H₂₄N₄O₂BrF | Requires | C 55.6 | H 5.1 | N 11.8% |

### Process (c)

A suspension of 4-chloro-6-methoxy-7-(1-methylpiperidin-4-ymethoxy)quinazoline (200mg, 0.62mmol) and 4-bromo-2-fluoroaniliue (142mg, 0.74mmol) in isopropanol (3ml) containing 6N hydrogen chloride in isopropanol (110µl, 0.68ml) was heated at reflux for 1.5 hours. After cooling, the precipitate was collected by filtration, washed with isopropanol followed by ether and dried under vacuum to give 4-(4-bromo-2-fluoroanilino)-6-methoxy-7-(1-methylpiperidin-4-ymethoxy)quinazoline hydrochloride (304mg, 90%).

| | | | | |
|---|---|---|---|---|
| Elemental analysis: | Found | C 47.9 | H 4.9 | N 10.0 |
| C₂₂H₂₄N₄O₂BrF 0.5H₂O 1.8HCl | Requires | C 48.2 | H 5.0 | N 10.1% |

0.08 isopropanol

The NMR spectrum of the protonated form of 4-(4-bromo-2-fluoroanilino)-6-methoxy-7-(1-methylpiperidin-4-ylmethoxy)quinazoline hydrochloride shows the presence of two forms A and B in a ratio A:B of approximately 9:1.
¹H NMR Spectrum: (DMSOd₆) 1.6-1.78 (m, form A 2H); 1.81-1.93 (br s, form B 4H); 1.94-2.07 (d, form A 2H); 2.08-2.23 (br s, form A 1H); 2.29-2.37 (br s, form B 1H); 2.73 (d, form A 3H); 2.77 (d, form B 3H); 2.93-3.10 (q, form A 2H); 3.21 (br s, form B 2H); 3.27 (br s, form B 2H); 3.42-3.48 (d, form A 2H); 4.04 (s, 3H); 4.10 (d, form A 2H); 4.29 (d, form B 2H); 7.49 (s, 1H) ; 7.53-7.61 (m, 2H); 7.78 (d, 1H); 8.47 (s, 1H); 8.81 (s, 1H); 10.48 (br s, form A 1H); 10.79 (br s, form B 1H); 11.90 (br s, 1H)

For another NMR reading, some solid potassium carbonate was added into the DMSO solution of the 4-(4-bromo-2-fluoroanilino)-6-methoxy-7-(1-methylpiperidin-4-ylmethoxy)quinazoline hydrochloride described above, in order to release the free base in the NMR tube. The NMR spectrum was then recorded again and showed only one form as described below:
¹H NMR Spectrum: (DMSOd₆; solid potassium carbonate) 1.3-1.45 (m, 2H) ; 1.75 (d, 2H) ; 1.7-1.9(m, 1H) ; 1.89 (t, 2H) ; 2.18 (s, 3H) ; 2.8 (d, 2H) ; 3.98 (s, 3H) ; 4.0 (d, 2H) ; 7.2 (s, 1H) ; 7.48 (d, 1H) ; 7.55 (t, 1H) ; 7.68 (d, 1H) ; 7.8 (s, 1H) ; 8.35 (s, 1H) ; 9.75 (s, 1H)

A sample of 4-(4-bromo-2-fluoroanilino)-6-methoxy-7-(1-methylpiperidin-4-ylmethoxy)quinazoline (free base) was generated from the 4-(4-bromo-2-fluoroanilino)-6-methoxy-7-(1-methylpiperidin-4-ymethoxy)quinazoline hydrochloride, (prepared as described above), as follows:
4-(4-Bromo-2-fluoroanilino)-6-methoxy-7-(1-methylpiperidin-4-ylmethoxyquinazoline hydrochloride (50mg) was suspended in methylene chloride (2ml) and was washed with saturated sodium hydrogen carbonate. The methylene chloride solution was dried (MgSO₄) and the volatiles were removed by evaporation to give 4-(4-bromo-2-fluoroanilino)-6-methoxy-7-(1-methlylpiperidin-4-ylmethoxy)quinazoline (free base). The NMR of the free base so generated shows only one form as described below:
   ¹H NMR Spectrum: (DMSOd₆) 1.3-1.45 (m, 2H) ; 1.76 (d, 2H) ; 1.7-1.9(m, 1H) ; 1.9 (t, 2H) ; 2.19 (s, 3H) ; 2.8 (d, 2H) ; 3.95 (s, 3H) ; 4.02 (d, 2H) ; 7.2 (s, 1H) ; 7.48 (d, 1H) ; 7.55 (t, 1H) ; 7.68 (dd, 1H) ; 7.8 (s, 1H) ; 8.38 (s, 1H) ; 9.55(br s, 1H)

For another NMR reading, some CF₃COOD was added into the NMR DMSO solution of the 4-(4-bromo-2-fluoroanilino)-6-methoxy-7-(1-methylpiperidin-4-ylmethoxy)quinazoline (free base) described above and the NMR spectrum was recorded again. The spectrum of the protonated form of the 4-(4-bromo-2-fluoroanilino)-6-methoxy-7-(1-methylpiperidin-4-ylmethoxy)quinazoline trifluoroacetate salt so obtained shows the presence of two forms A and B in a ratio A: B of approximately 9:1.
¹H NMR Spectrum: (DMSOd₆; CF₃COOD) 1.5-1.7 (m, form A 2H); 1.93 (br s, form B 4H); 2.0-2.1 (d, form A 2H); 2.17 (br s, form A 1H); 2.35 (br s, form B1H); 2.71 (s, form A 3H); 2.73 (s, form B 3H); 2.97-3.09 (t, form A 2H); 3.23 (br s, form B 2H); 3.34 (br s, form B 2H); 3.47-3.57 (d, form A 2H); 4.02 (s, 3H); 4.15 (d, form A 2H); 4.30 (d, form B 2H); 7.2 (s, 1H); 7.3-7.5 (m, 2H); 7.6 (d, 1H); 7.9 (s, 1H); 8.7 (s, 1H)

The starting material was prepared as follows:
1-(*tert-*Butoxycarbonyl)-4-(4-methylphenylsulphonyloxymethyl)piperidine (40g, 0.11mol), (prepared as described for the starting material in process (a) above), was added to a suspension of ethyl 4-hydroxy-3-methoxybenzoate (19.6g, 0.1mol) and potassium carbonate (28g, 0.2mol) in dry DMF (200ml). After stirring at 95°C for 2.5 hours, the mixture was cooled to ambient temperature and partitioned between water and ethyl acetate/ether. The organic layer was washed with water, brine, dried (MgSO₄) and evaporated. The resulting oil was crystallised from petroleum ether and the suspension was stored overnight at 5°C. The solid was collected by filtration, washed with petroleum ether and dried under vacuum to give ethyl 4-(1-(*tert-*butoxycarbonyl)piperidin-4-ylmethoxy)-3-methoxybenzoate (35g, 89%).
m.p. 81-83°C
MS (ESI:): 416 [MNa]⁺
¹H NMR Spectrum: (CDCl₃) 1.2-1.35(m, 2H); 1.4(t, 3H); 1.48(s, 9H); 1.8-1.9(d, 2H); 2.0-2.15(m, 2H); 2.75(t, 2H); 3.9(d, 2H); 3.95(s, 3H); 4.05-4.25(br s, 2H); 4.35(q, 2H); 6.85(d, 1H); 7.55(s, 1H); 7.65(d, 1H)

| | | | | |
|---|---|---|---|---|
| Elemental analysis: | Found | C 63.4 | H 8.0 | N 3.5 |
| C₂₁H₃₁NO₆ 0.3H₂O | Requires | C 63.2 | H 8.0 | N 3.5% |

Formaldehyde (12M, 37% in water, 35ml, 420mmol) was added to a solution of ethyl 4-(1-(*tert-*butoxycarbonyl)piperidin-4-ylmethoxy)-3-methoxybenzoate (35g, 89mmol) in formic acid (35ml). After stirring at 95°C for 3 hours, the volatiles were removed by evaporation. The residue was dissolved in methylene chloride and 3M hydrogen chloride in ether (40ml, 120mmol) was added. After dilution with ether, the mixture was triturated until a solid was formed. The solid was collected by filtration, washed with ether and dried under vacuum overnight at 50°C to give ethyl 3-methoxy-4-(1-methylpiperidin-4-ylmethoxy)benzoate (30.6g, quant.).
MS (ESI): 308 [MH]⁺ +
¹H NMR Spectrum: (DMSOd₆) 1.29(t, 3H); 1.5-1.7(m, 2H); 1.95(d, 2H); 2.0-2.15(br s, 1H); 2.72(s, 3H); 2.9-3.1(m, 2H); 3.35-3.5(br s, 2H); 3.85(s, 3H); 3.9-4.05(br s, 2H); 4.3(q, 2H); 7.1(d, 1H); 7.48(s, 1H); 7.6(d, 1H)

A solution of ethyl 3-methoxy-4-(1-methylpiperidin-4-ylmethoxy)benzoate (30.6g, 89mmol) in methylene chloride (75ml) was cooled to 0-5°C. TFA (37.5ml) was added followed by the dropwise addition over 15 minutes of a solution of fuming 24N nitric acid (7.42ml, 178mmol) in methylene chloride (15ml). After completion of the addition, the solution was allowed to warm up and stirred at ambient temperature for 2 hours. The volatiles were removed under vacuum and the residue was dissolved in methylene chloride (50ml). The solution was cooled to 0-5°C and ether was added. The precipitate was collected by filtration, and dried under vacuum at 50°C. The solid was dissolved in methylene chloride (500ml) and 3M hydrogen chloride in ether (30ml) was added followed by ether (500ml). The solid was collected by filtration and dried under vacuum at 50°C to give ethyl 3-methoxy-4-(1-methylpiperidin-4-ylmethoxy)-6-nitrobenzoate (28.4g, 82%).
MS (ESI): 353 [MH]⁺
¹H NMR Spectrum: (DMSOd₆) 1.3(t, 3H); 1.45-1.65(m, 2H); 1.75-2.1(m, 3H); 2.75(s, 3H); 2.9-3.05(m, 2H); 3.4-3.5(d, 2H); 3.95(s, 3H); 4.05(d, 2H); 4.3(q, 2H); 7.32(s, 1H); 7.66(s, 1H)

A suspension of ethyl 3-methoxy-4-(1-methylpiperidin-4-ylmethoxy)-6-nitrobenzoate (3.89g, 10mmol) in methanol (80ml) containing 10% platinum on activated carbon (50% wet) (389mg) was hydrogenated at 1.8 atmospheres pressure until uptake of hydrogen ceased. The mixture was filtered and the filtrate was evaporated. The residue was dissolved in water (30ml) and adjusted to pH10 with a saturated solution of sodium hydrogen carbonate. The mixture was diluted with ethyl acetate/ether (1/1) and the organic layer was separated. The aqueous layer was further extracted with ethyl acetate/ether and the organic layers were combined. The organic layers were washed with water, brine, dried (MgSO₄), filtered and evaporated. The resulting solid was triturated in a mixture of ether/petroleum ether, filtered, washed with petroleum ether and dried under vacuum at 60°C to give ethyl 6-amino-3-methoxy-4-(1-methylpiperidin-4-ylmethoxy)benzoate (2.58g, 80%).
m.p. 111-112°C
MS (ESI): 323 [MH]⁺
¹H NMR Spectrum: (CDCl₃) 1.35(t, 3H); 1.4-1.5(m, 2H); 1.85(m, 3H); 1.95(t, 2H); 2.29(s, 3H); 2.9(d, 2H); 3.8(s, 3H); 3.85(d, 2H); 4.3(q, 2H); 5.55(br s, 2H); 6.13(s, 1H); 7.33(s, 1H)

| | | | | |
|---|---|---|---|---|
| Elemental analysis: | Found | C 62.8 | H 8.5 | N 8.3 |
| C₁₇H₂₆N₂O₄ 0.2H₂O | Requires | C 62.6 | H 8.2 | N 8.6% |

A solution of ethyl 6-amino-3-methoxy-4-(1-methylpiperidin-4-ylmethoxy)benzoate (16.1g, 50mmol) in 2-methoxyethanol (160ml) containing formamidine acetate (5.2g, 50mmol) was heated at 115°C for 2 hours. Formamidine acetate (10.4g, 100mmol) was added in portions every 30 minutes over 4 hours. Heating was prolonged for 30 minutes after the last addition. After cooling, the volatiles were removed under vacuum. The solid was dissolved in ethanol (100ml) and methylene chloride (50ml). The precipitate was removed by filtration and the filtrate was concentrated to a final volume of 100ml. The suspension was cooled to 5°C and the solid was collected by filtration, washed with cold ethanol followed by ether and dried under vacuum overnight at 60°C to give 6-methoxy-7-(1-methylpiperidin-4-ylmethoxy)-3,4-dihydroquinazolin-4-one (12.7g, 70%).
MS (ESI): 304 [MH]⁺
¹H NMR Spectrum: (DMSOd₆) 1.25-1.4(m, 2H); 1.75(d, 2H); 1.9(t, 1H); 1.9(s, 3H); 2.16(s, 2H); 2.8(d, 2H); 3.9(s, 3H); 4.0(d, 2H); 7.11(s, 1H); 7.44(s, 1H); 7.97(s, 1H)

A solution of 6-methoxy-7-(1-methylpiperidin-4-ylmethoxy)-3,4-dihydroquinazolin-4-one (2.8g, 9.24mmol) in thionyl chloride (28ml) containing DMF (280µl) was heated at reflux at 85°C for 1 hour. After cooling, the volatiles were removed by evaporation. The precipitate was triturated with ether, filtered, washed with ether and dried under vacuum The solid was dissolved in methylene chloride and saturated aqueous sodium hydrogen carbonate was added. The organic layer was separated, washed with water, brine, dried (MgSO₄) and evaporated to give 4-chloro-6-methoxy-7-(1-methylpiperidin-4-ymethoxy)quinazoline (2.9g, 98%).
MS (ESI): 322 [MH]⁺
¹H NMR Spectrum: (DMSOd₆) 1.3-1.5(m, 2H); 1.75-1.9(m, 3H); 2.0(t, 1H); 2.25(s, 3H); 2.85(d, 2H); 4.02(s, 3H); 4.12(d, 2H); 7.41(s, 1H); 7.46(s, 1H); 8.9(s, 1H)

Alternatively, the 6-methoxy-7-(1-methylpiperidin-4-ylmethoxy)-3,4-dihydroquinazolin-4-one can be prepared as follows:
Sodium hydride (1.44g of a 60% suspension in mineral oil, 36mmol) was added in portions over 20 minutes to a solution of 7-benzyloxy-6-methoxy-3,4-dihydroquinazolin-4-one (8.46g, 30mmol), (prepared, for example, as described in WO 97/22596, Example 1), in DMF (70ml) and the mixture was stirred for 1.5 hours. Chloromethyl pivalate (5.65g, 37.5mmol) was added in portions and the mixture stirred for 2 hours at ambient temperature. The mixture was diluted with ethyl acetate (100ml) and poured onto ice/water (400ml) and 2N hydrochloric acid (4ml). The organic layer was separated and the aqueous layer extracted with ethyl acetate, the combined extracts were washed with brine, dried (MgSO₄) and the solvent removed by evaporation. The residue was triturated with a mixture of ether and petroleum ether, the solid was collected by filtration and dried under vacuum to give 7-benzyloxy-6-methoxy-3-((pivaloyloxy)methyl)-3,4-dihydroquinazolin-4-one (10g, 84%).
   ¹H NMR Spectrum: (DMSOd₆) 1.11(s, 9H); 3.89(s, 3H); 5.3(s, 2H); 5.9(s, 2H); 7.27(s, 1H); 7.35(m, 1H); 7.47(t, 2H); 7.49(d, 2H); 7.51(s, 1H); 8.34(s, 1H)

A mixture of 7-benzyloxy-6-methoxy-3-((pivaloyloxy)methyl)-3,4-dihydroquinazolin-4-one (7g, 17.7mmol) and 10% palladium-on-charcoal catalyst (700mg) in ethyl acetate (250ml), DMF (50ml), methanol (50ml) and acetic acid (0.7ml) was stirred under hydrogen at atmospheric pressure for 40 minutes. The catalyst was removed by filtration and the solvent removed from the filtrate by evaporation. The residue was triturated with ether, collected by filtration and dried under vacuum to give 7-hydroxy-6-methoxy-3-((pivaloyloxy)methyl)-3,4-dihydroquinazolin-4-one (4.36g, 80%).
¹H NMR Spectrum: (DMSOd₆) 1.1(s, 9H); 3.89(s, 3H); 5.89(s, 2H); 7.0(s, 1H); 7.48(s, 1H); 8.5(s, 1H)

Triphenylphosphine (1.7g, 6.5mmol) was added under nitrogen to a suspension of 7-hydroxy-6-methoxy-3-((pivaloyloxy)methyl)-3,4-dihydroquinazolin-4-one (1.53g, 5mmol) in methylene chloride (20ml), followed by the addition of 1-(*tert-*butoxycarbonyl)-4-(hydroxymethyl)piperidine (1.29g, 6mmol), (prepared as described for the starting material in process (a) above), and by a solution of diethyl azodicarboxylate (1.13g, 6.5mmol) in methylene chloride (5ml). After stirring for 30 minutes at ambient temperature, the reaction mixture was poured onto a column of silica and was eluted with ethyl acetate/petroleum ether (1/1 followed by 6/5, 6/4 and 7/3). Evaporation of the fractions containing the expected product led to an oil that crystallised following trituration with pentane. The solid was collected by filtration and dried under vacuum to give 7-(1-(*tert-*butoxycarbonyl)piperidin-4-ylmethoxy)-6-methoxy-3-((pivaloyloxy)methyl)-3,4-dihydroquinazolin-4-one (232g, 92%).
MS - ESI: 526 [MNa]⁺
¹H NMR Spectrum: (CDCl₃) 1.20 (s, 9H), 1.2-1.35 (m, 2H), 1.43 (s, 9H), 1.87 (d, 2H), 2.05-2.2 (m, 1H), 2.75 (t, 2H), 3.96 (d, 2H), 3.97 (s, 3H), 4.1-4.25 (br s, 2H), 5.95 (s, 2H), 7.07 (s, 1H), 7.63 (s, 1H), 8.17 (s, 1H)

| | | | | |
|---|---|---|---|---|
| Elemental analysis: | Found | C 61.8 | H 7.5 | N 8.3 |
| C₂₆H₃₇N₃O₇ | Requires | C 62.0 | H 7.4 | N 8.3% |

A solution of 7-(1-(*tert-*butoxycarbonyl)piperidin-4-ylmethoxy)-6-methoxy-3-((pivaloyloxy)methyl)-3,4-dihydroquinazolin-4-one (2.32g, 4.6mmol) in methylene chloride (23ml) containing TFA (5ml) was stirred at ambient temperature for 1 hour. The volatiles were removed under vacuum. The residue was partitioned between ethyl acetate and sodium hydrogen carbonate. The organic solvent was removed under vacuum and the residue was filtered. The precipitate was washed with water, and dried under vacuum. The solid was azeotroped with toluene and dried under vacuum to give 6-methoxy-7-(piperidin-4-ylmethoxy)-3-((pivaloyloxy)methyl)-3,4-dihydroquinazolin-4-one (1.7g, 92%).
MS - ESI: 404 [MH]⁺
¹H NMR Spectrum: (DMSOd₆; CF₃COOD) 1.15 (s, 9H), 1.45-1.6 (m, 2H), 1.95 (d, 2H), 2.1-2.25 (m, 1H), 2.95 (t, 2H), 3.35 (d, 2H), 3.95 (s, 3H), 4.1 (d, 2H), 5.95 (s, 2H), 7.23 (s, 1H), 7.54 (s, 1H), 8.45 (s, 1H)

A 37% aqueous solution of formaldehyde (501µl, 6mmol) followed by sodium cyanoborohydride (228mg, 3.6mmol) were added in portions to a solution of 6-methoxy-7-(piperidin-4-ylmethoxy)-3-((pivaloyloxy)methyl)-3,4-dihydroquinazolin-4-one (1.21g, 3mmol) in a mixture of THF/methanol (10ml/10ml). After stirring for 30 minutes at ambient temperature, the organic solvents were removed under vacuum and the residue was partitioned between methylene chloride and water. The organic layer was separated, washed with water and brine, dried (MgSO₄) and the volatiles were removed by evaporation. The residue was triturated with ether and the resulting solid was collected by filtration, washed with ether and dried under vacuum to give 6-methoxy-7-(1-methylpiperidin-4-ylmethoxy)-3-((pivaloyloxy)methyl)-3,4-dihydroquinazolin-4-one (1.02g, 82%).
MS - ESI: 418 [MH]⁺
¹H NMR Spectrum: (CDCl₃) 1.19 (s, 9H), 1.4-1.55 (m, 2H), 1.9 (d, 2H), 2.0 (t, 2H), 1.85-2.1 (m, 1H), 2.3 (s, 3H), 2.92 (d, 2H), 3.96 (s, 3H), 3.99 (d, 2H), 5.94 (s, 2H), 7.08 (s, 1H), 7.63 (s, 1H), 8.17 (s, 1H)

A saturated solution of ammonia in methanol (14ml) was added to a solution of 6-methoxy-7-(1-methylpiperidin-4-ylmethoxy)-3-((pivaloyloxy)methyl)-3,4-dihydroquinazolin-4-one (1.38g, 3.3mmol) in methanol (5ml). After stirring for 20 hours at ambient temperature, the suspension was diluted with methylene chloride (10ml). The solution was filtered. The filtrate was evaporated under vacuum and the residue was triturated with ether, collected by filtration, washed with ether and dried under vacuum to give 6-methoxy-7-(1-methylpiperidin-4-ylmethoxy)-3,4-dihydroquinazolin-4-one (910mg, 83%).
MS - ESI: 304 [MH]⁺
¹H NMR Spectrum: (DMSOd₆) 1.3-1.45 (m, 2H), 1.75 (d, 2H), 1.7-1.85 (m, 1H), 1.9 (t, 2H), 2.2 (s, 3H), 2.8 (d, 2H), 3.9 (s, 3H), 4.0 (d, 2H), 7.13 (s, 1H), 7.45 (s, 1H), 7.99 (s, 1H)

The following tests were used to demonstrate the activity of ZD6474 in combination with 5-FU and CPT-11.

### Human LS-174T colon tumour xenografts in Nude mice

10⁷ LS-174T tumour cells in 0.2 ml of serum free Roswell Park Memorial Institute (RPMI)- 1640 medium were injected subcutaneously (s.c.) into the flanks of 10 athymic (*nu*/*nu* genotype) mice. When tumour sizes reached 700-1000 mm³ (3-4 weeks), tumours were surgically excised and smaller tumour fragments thereof (20-30 mg) were implanted s.c. in the right flank of 120 *Nude* mice. When tumours reached a volume of 100 to 200 mm³ (14-16 days after the graft), mice were randomized into groups (13 - 15 per group) and treatment started.

### (a) 5-FU + ZD6474

o The control group (Group 1) received a daily oral (p.o.) administration of ZD6474 vehicle for 14 consecutive days (day 0 - 13) combined with two intravenous (i.v.) injections of saline (the vehicle for 5-FU) on day 0 and 7.
o For Group 2, the treatments consisted of a daily p.o. administration of ZD6474 alone at 25mg/kg/adminstration for 14 consecutive days (day 0 - 13) combined with two i.v. injections of saline (the vehicle for 5-FU) on day 0 and 7. ZD6474 was prepared as a suspension in 1% polysorbate 80 (i.e. a 1% (v/v) solution of polyoxyethylene (20) sorbitan mono-oleate in deionised water).
□ Group 3 received two i.v. injections of 5-FU at 75mg/kg/injection, on day 0 and 7, combined with a daily p.o. administration of ZD6474 vehicle for 14 consecutive days (day 0-13).
□ Group 4 received daily p.o. administration of ZD6474 at 25mg/kg/adminstration for 14 consecutive days (day 0 - 13) combined with two i.v. injections of 5-FU at 75mg/kg/injection, on day 0 and 7.

The administration volume of ZD6474 was 10.0 ml/kg (200 µl for a 20 g mouse). The injection volume of 5-FU was 10.0 ml/kg (200 µl for a 20 g mouse).

| **Group** | **Treatments** | **Combined drug doses (mg base/kg/inj.)** | **Adm. route** | **No. Treatments** | **No. Treatment /day** | **Days-interval between treatment (Days)** |
|---|---|---|---|---|---|---|
| **1** | Vehicles of ZD6474 and 5-FU | 0.0 | p.o. for ZD6474 | 14 p.o. | 1 p.o. | 1 for p.o. |
| | | | vehicle i.v. for saline | 2 i.v. | 1 i.v. | 7 for i.v. |
| **2** | ZD6474 + saline | 25.0 | p.o. for ZD6474 | 14 p.o. | 1 p.o. | 1 for p.o. |
| | | | i. v. for saline | 2 i. v. | 1 i. v. | 7 for i.v. |
| **3** | 5-FU + ZD6474 vehicle | 75.0 | i.v. for 5-FU | 14 p.o. | 1 p.o. | 1 for p.o. |
| | | | p.o. for ZD6474 vehicle | 2 i.v. | 1 i.v. | 7 for i.v. |
| **4** | ZD6474 + 5-FU | 25.0 for ZD6474 75.0 for 5-FU | p.o. for ZD6474 | 14 p.o. | 1 p.o. | 1 for p.o. |
| | | | i.v. for 5-FU | 2 i.v. | 1 i.v. | 7 for i.v. |

Tumor volumes (mm³) were assessed at least twice weekly by bilateral Vernier caliper measurement and, taking length to be the longest diameter across the tumor and width the corresponding perpendicular, calculated using the formula (π/6) x (length x width) x the square root of (length x width). Growth inhibition from the start of treatment was assessed by comparison of the differences in tumor volume between control and treated groups. For all mice, the study was stopped when tumours reached 2,000 mm³. For all mice, the tumours were excised and weights recorded upon termination of the study.

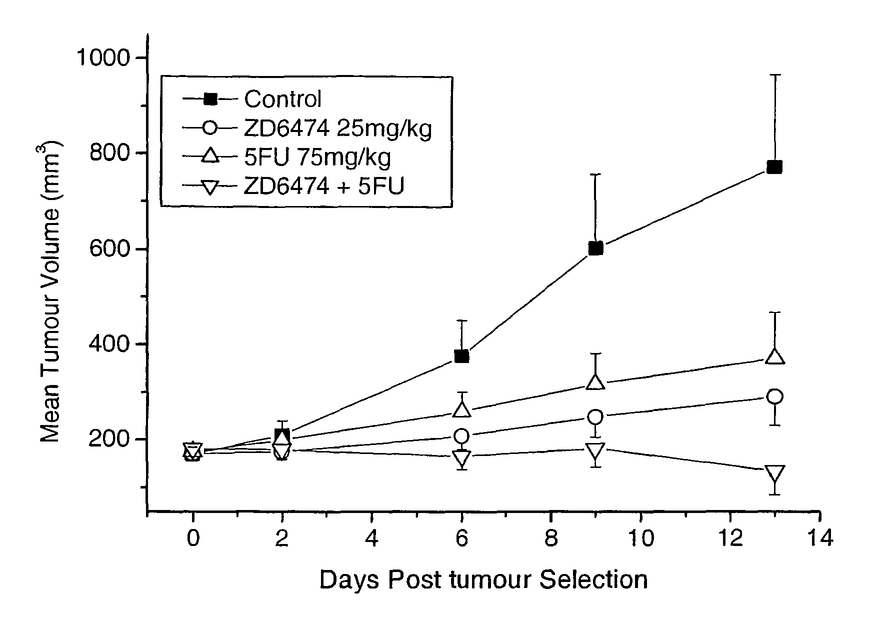

| Treatment | Inhibition of Control Tumour Growth at day 13 | P value (one-tailed two-sample t-test) | Regressions* |
|---|---|---|---|
| | | | |
| ZD6474 (25mg/kg/day p.o., d 0 -13) | 80 % | 0.01 | 5/15 |
| 5-FU (75 mg/kg i.v., d 0 and 7) | 68 % | 0.03 | 6/15 |
| ZD6474 + 5-FU | 107 % (Regression) | 0.002 | 8/13 |

| | | | |
|---|---|---|---|
| * Number of tumours which had regressed by ≥ 10% in volume by day 13, when compared with their pre-treatment volume on day 0. | | | |

The combination of 5-FU with ZD6474 produced a significantly greater inhibition of tumour growth than 5-FU alone (P= 0.018 at day 13, by one-tailed two-sample t test).

The combination of 5-FU with ZD6474 produced a significantly greater inhibition of tumour growth than ZD6474 alone, (P= 0.027 at day 13, by one-tailed two-sample t test).

The combination of 5-FU with ZD6474 produced more tumour regressions (62%) than ZD6474 alone (33%) or 5-FU alone (40%).

### (b) CPT-11 + ZD6474

□ The control group (Group 1) received a daily oral (p.o.) administration of ZD6474 vehicle for 14 consecutive days (day 0 -13) combined with two intravenous (i.v.) injections of saline (the vehicle for CPT-11) on day 0 and 7. The control group was not continued past this period, as some of the tumour volumes were considered too large (~ 2 cm³).
□ For Group 2, the treatments consisted of a daily p.o. administration of ZD6474 alone at 25mg/kg/adminstration for 21 consecutive days (day 0 - 20) combined with three i.v. injections of saline (the vehicle for CPT-11) on day 0, 7 and 14. ZD6474 was prepared as a suspension in 1% polysorbate 80 (i.e. a 1% (v/v) solution of polyoxyethylene (20) sorbitan mono-oleate in deionised water).
□ Group 3 received three i.v. injections of CPT-11 at 20mg/kg/injection, on day 0, 7 and 14, combined with a daily p.o. administration of ZD6474 vehicle for 21 consecutive days (day 0 - 20).
□ Group 4 received daily p.o. administration of ZD6474 at 25mg/kg/adminstration for 21 consecutive days (day 0 - 20) combined with three i.v. injections of CPT-11 at 20mg/kg/injection, on day 0, 7 and 14.

The administration volume of ZD6474 was 10.0 ml/kg (200 µl for a 20 g mouse). The injection volume of CPT-11 was 10.0 ml/kg (200 µl for a 20 g mouse).

| **Group** | **Treatments** | **Combined drug doses (mg base/kg/inj.)** | **Adm. route** | **No. Treatments** | **No. Treatment /day** | **Days-interval between treatment (Days)** |
|---|---|---|---|---|---|---|
| **1** | Vehicles of ZD6474 and CPT-11 | 0.0 | p.o. for ZD6474 vehicle | 14 p.o. | 1 p.o. | 1 for p.o. |
| | | | | 2 i.v. | 1 i. v. | 7 for i. v. |
| | | | i.v. for saline | | | |
| **2** | ZD6474 + saline | 25.0 | p.o. for ZD6474 | 21 p.o. | 1 p.o. | 1 for p.o. |
| | | | | 3 i.v. | 1 i.v. | 7 for i.v. |
| | | | i.v. for saline | | | |
| **3** | CPT-11 + ZD6474 vehicle | 20.0 | i.v. for CPT-11 | 21 p.o. | 1 p.o. | 1 for p.o. |
| | | | p.o. for ZD6474 vehicle | 3 i.v. | 1 i.v. | 7 for i.v. |
| **4** | ZD6474 + CPT-11 | 25.0 for ZD6474 | p.o. for | 21 p.o. | 1 p.o. | 1 for p.o. |
| | | 20.0 for CPT-11 | ZD6474 | 3 i.v. | 1 i.v. | 7 for i.v. |
| | | | i. v. for CPT-11 | | | |

Tumor volumes (mm³) were assessed at least twice weekly by bilateral Vernier caliper measurement and, taking length to be the longest diameter across the tumor and width the corresponding perpendicular, calculated using the formula (π/6) x (length x width) x square root of (length x width). Growth inhibition from the start of treatment was assessed by comparison of the differences in tumor volume between control and treated groups. For all mice, the study was stopped when tumours reached 2,000 mm³. For all mice, the tumours were excised and weights recorded upon termination of the study.

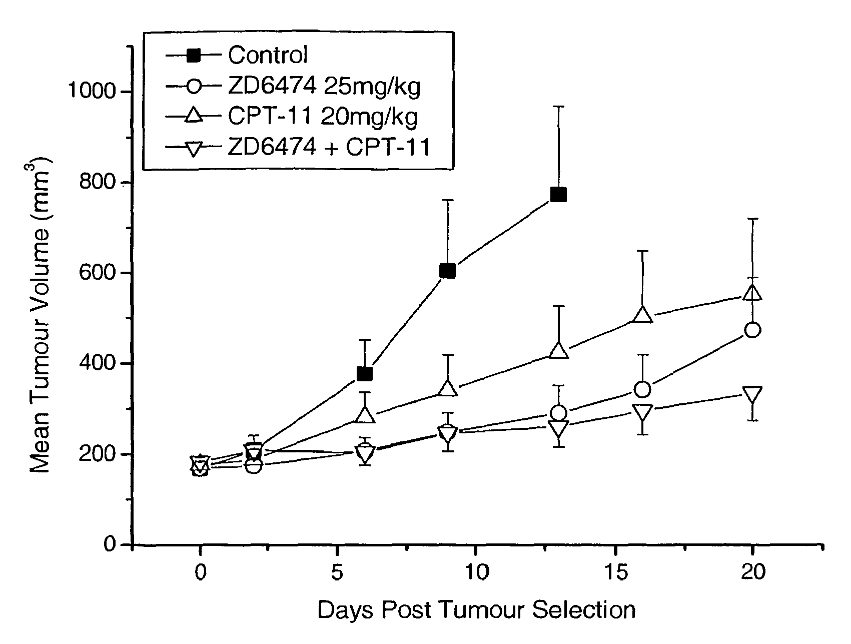

The mean tumour volumes on day 20 for mice treated with ZD6474 (25mg/kg/day p.o., d 0 - 20), CPT-11 (20 mg/kg i.v., d 0, 7 and 14), or the combination thereof, were 475mm³, 552mm³ and 336mm³ respectively.

An analogous experiment may be used to look at the combination of ZD6474, 5-FU and CPT-11 in this animal model.

An analogous experiment may be used to look at the combination of ZD6474, 5-FU, CPT-11 and ionising radiation in this animal model.

## Claims

1. Use of 4-(4-bromo-2-fluoroanilino)-6-methoxy-7-(1-methylpiperidin-4-ylmethoxy)quinazoline, also known as ZD6474: 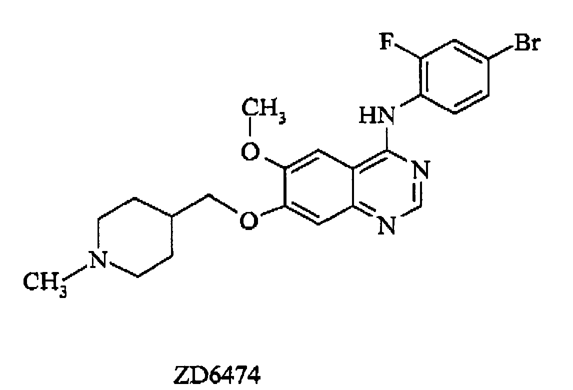 or a pharmaceutically acceptable salt thereof and one of:
a) 5-FU;
b) CPT-11; and
c) 5-FU and CPT-11
in the manufacture of a medicament for the treatment of cancer in a warm-blooded animal such as a human.

2. Use according to Claim 1 wherein the cancer involves a solid tumour.

3. Use of ZD6474 or a pharmaceutically acceptable salt thereof and 5-FU in the manufacture of a medicament for use in the treatment of cancer in a warm-blooded animal such as a human which is being treated with ionising radiation.

4. Use of ZD6474 or a pharmaceutically acceptable salt thereof and CPT-11 in the manufacture of a medicament for use in the treatment of cancer in a warm-blooded animal such as a human which is being treated with ionising radiation.

5. Use of ZD6474 or a pharmaceutically acceptable salt thereof and 5-FU and CPT-11 in the manufacture of a medicament for use in the treatment of cancer in a warm-blooded animal such as a human which is being treated with ionising radiation.

6. Use according to any one of Claims 3 to 5 wherein the cancer involves a solid tumour.

7. Use according to any one of the preceding claims wherein the cancer is colorectal cancer.

8. A pharmaceutical composition comprising ZD6474 or a pharmaceutically acceptable salt thereof, and 5-FU in association with a pharmaceutically acceptable excipient or carrier.

9. A pharmaceutical composition comprising ZD6474 or a pharmaceutically acceptable salt thereof, and CPT-11 in association with a pharmaceutically acceptable excipient or carrier,

10. A pharmaceutical composition comprising ZD6474 or a pharmaceutically acceptable salt thereof, and 5-FU and CPT-11 in association with a pharmaceutically acceptable excipient or carrier.

11. A kit comprising ZD6474 or a pharmaceutically acceptable salt thereof, and 5-FU.

12. A kit comprising ZD6474 or a pharmaceutically acceptable salt thereof, and CPT-11.

13. A kit comprising ZD6474 or a pharmaceutically acceptable salt thereof, and 5-FU and CPT-11.

## Patentansprüche

1. Verwendung von 4-(4-Brom-2-fluoranilino)-6-methoxy-7-(1-methylpiperidin-4-ylmethoxy)-chinazolin, das auch als ZD6474 bekannt ist: 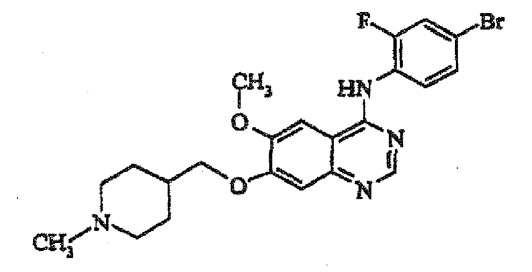 oder eines pharmazeutisch annehmbaren Salzes davon und
a) 5-FU;
b) CPT-11 oder
c) 5-FU und CPT-11
bei der Herstellung eines Arzneimittels zur Behandlung von Krebs bei einem Warmblüter wie einem Menschen.

2. Verwendung nach Anspruch 1, wobei bei der Krebserkrankung ein solider Tumor vorliegt.

3. Verwendung von ZD6474 oder einem pharmazeutisch annehmbaren Salz davon und 5-FU bei der Herstellung eines Arzneimittels zur Verwendung bei der Behandlung von Krebs bei einem Warmblüter wie einem Menschen, der mit ionisierender Strahlung behandelt wird.

4. Verwendung von ZD6474 oder einem pharmazeutisch annehmbaren Salz davon und CPT-11 bei der Herstellung eines Arzneimittels zur Verwendung bei der Behandlung von Krebs bei einem Warmblüter wie einem Menschen, der mit ionisierender Strahlung behandelt wird.

5. Verwendung von ZD6474 oder einem pharmazeutisch annehmbaren Salz davon und 5-FU und CPT-11 bei der Herstellung eines Arzneimittels zur Verwendung bei der Behandlung von Krebs bei einem Warmblüter wie einem Menschen, der mit ionisierender Strahlung behandelt wird.

6. Verwendung nach einem der Ansprüche 3 bis 5, wobei bei der Krebserkrankung ein solider Tumor vorliegt.

7. Verwendung nach einem der vorhergehenden Ansprüche, wobei es sich bei dem Krebs um Kolorektalkrebs handelt.

8. Pharmazeutische Zusammensetzung, die ZD6474 oder ein pharmazeutisch annehmbares Salz davon und 5-FU zusammen mit einem pharmazeutisch annehmbaren Hilfsstoff oder Träger enthält.

9. Pharmazeutische Zusammensetzung, die ZD6474 oder ein pharmazeutisch annehmbares Salz davon und CPT-11 zusammen mit einem pharmazeutisch annehmbaren Hilfsstoff oder Träger enthält.

10. Pharmazeutische Zusammensetzung, die ZD6474 oder ein pharmazeutisch annehmbares Salz davon und 5-FU und CPT-11 zusammen mit einem pharmazeutisch annehmbaren Hilfsstoff oder Träger enthält.

11. Kit, enthaltend ZD6474 oder ein pharmazeutisch annehmbares Salz davon und 5-FU.

12. Kit, enthaltend ZD6474 oder ein pharmazeutisch annehmbares Salz davon und CPT-11.

13. Kit, enthaltend ZD6474 oder ein pharmazeutisch annehmbares Salz davon und 5-FU und CPT-11.

## Revendications

1. Utilisation de la 4-(4-bromo-2-fluoroanilino)-6-méthoxy-7-(1-méthylpipéridin-4-ylméthoxy)quinazoline, également appelée ZD6474 : 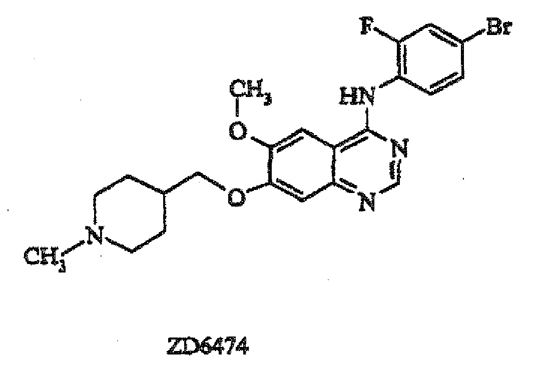 ou d'un sel pharmaceutiquement acceptable de celle-ci et l'un de :
a) 5-FU ;
b) CPT-11 ; et
c) 5-FU et CPT-11
dans la fabrication d'un médicament destiné au traitement du cancer chez un animal à sang chaud tel que l'homme.

2. Utilisation selon la revendication 1, **caractérisée en ce que** le cancer comprend une tumeur solide.

3. Utilisation de ZD6474 ou d'un sel pharmaceutiquement acceptable de celui-ci et de 5-FU dans la fabrication d'un médicament destiné à être utilisé dans le traitement du cancer chez un animal à sang chaud tel que l'homme recevant un traitement par des radiations ionisantes.

4. Utilisation de ZD6474 ou d'un sel pharmaceutiquement acceptable de celui-ci et de CPT-11 dans la fabrication d'un médicament destiné à être utilisé dans le traitement du cancer chez un animal à sang chaud tel que l'homme recevant un traitement par des radiations ionisantes.

5. Utilisation de ZD6474 ou d'un sel pharmaceutiquement acceptable de celui-ci et de 5-FU et de CPT-11 dans la fabrication d'un médicament destiné à être utilisé dans le traitement du cancer chez un animal à sang chaud tel que l'homme recevant un traitement par des radiations ionisantes.

6. Utilisation selon l'une quelconque des revendications 3 à 5, **caractérisée en ce que** le cancer comprend une tumeur solide.

7. Utilisation selon l'une quelconque des revendications précédentes, **caractérisée en ce que** le cancer est le cancer colorectal.

8. Composition pharmaceutique comprenant le ZD6474 ou un sel pharmaceutiquement acceptable de celui-ci, et le 5-FU en association avec un excipient ou un support pharmaceutiquement acceptable.

9. Composition pharmaceutique comprenant le ZD6474 ou un sel pharmaceutiquement acceptable de celui-ci, et le CPT-11 en association avec un excipient ou un support pharmaceutiquement acceptable.

10. Composition pharmaceutique comprenant le ZD6474 ou un sel pharmaceutiquement acceptable de celui-ci, et le 5-FU et le CPT-11 en association avec un excipient ou un support pharmaceutiquement acceptable.

11. Kit comprenant le ZD6474 ou un sel pharmaceutiquement acceptable de celui-ci et le 5-FU.

12. Kit comprenant le ZD6474 ou un sel pharmaceutiquement acceptable de celui-ci et le CPT-11.

13. vit comprenant le ZD6474 ou un sel pharmaceutiquement acceptable de celui-ci, et le 5-FU et le CPT-11.
